(19) Europäisches Patentamt
European Patent Office
Office européen des brevets

(11) EP 4 617 281 A1

(12) EUROPEAN PATENT APPLICATION
published in accordance with Art. 153(4) EPC

(43) Date of publication:
**17.09.2025 Bulletin 2025/38**

(21) Application number: **23888130.4**

(22) Date of filing: **10.11.2023**

(51) International Patent Classification (IPC):
***C07K 1/107*** *(2006.01)* ***C07K 14/54*** *(2006.01)*
***A61K 38/20*** *(2006.01)* ***A61K 47/60*** *(2017.01)*

(52) Cooperative Patent Classification (CPC):
**A61K 47/60; A61K 45/06; C07K 1/1077; C07K 14/5434;** A61K 38/00

(86) International application number:
**PCT/CN2023/131105**

(87) International publication number:
**WO 2024/099445 (16.05.2024 Gazette 2024/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.11.2022 CN 202211407900**

(71) Applicant: **Kanglitai Biomedical (Qingdao) Co., Ltd.**
**Qingdao, Shandong 266111 (CN)**

(72) Inventors:
- **WANG, Jiangang**
  **Qingdao, Shandong 266111 (CN)**
- **QIAO, Tiankui**
  **Qingdao, Shandong 266111 (CN)**
- **ZHENG, Binbin**
  **Qingdao, Shandong 266111 (CN)**
- **LI, Tingting**
  **Qingdao, Shandong 266111 (CN)**
- **LIU, Yi**
  **Qingdao, Shandong 266111 (CN)**
- **LI, Lei**
  **Qingdao, Shandong 266111 (CN)**
- **QIAO, Lei**
  **Qingdao, Shandong 266111 (CN)**
- **WU, Mingyuan**
  **Qingdao, Shandong 266111 (CN)**

(74) Representative: **Herrero & Asociados, S.L.**
**Cedaceros, 1**
**28014 Madrid (ES)**

Remarks:
The complete document including Reference Table(s) and the Sequence Listing(s) can be downloaded from the EPO website

# (54) SITE-SPECIFIC PEGYLATED HETEROMERIC PROTEIN OR POLYPEPTIDE, AND PREPARATION METHOD THEREFOR AND USE THEREOF

(57) Provided is a site-specifically PEGylated heteromeric protein or polypeptide and a preparation method and use of the heteromeric protein or polypeptide. First, a method for site-specific PEGylation of a protein or polypeptide is provided. The method includes: providing a protein or polypeptide to be modified, the protein or polypeptide to be modified including a target N-terminal $\alpha$-amino group and a non-target N-terminal $\alpha$-amino group, and mutating and blocking the non-target N-terminal $\alpha$-amino group; and subjecting the target N-terminal $\alpha$-amino group to a site-specific modification reaction with an aldehyde or ketone derivative of polyethylene glycol to generate a site-specifically PEGylated protein or polypeptide. Heteromeric protein or polypeptide products subjected to site-specific single modification with polyethylene glycol by this method has a specific and controllable modification position, which can bring about a clearly extended half-life and biased composition activity, and can be better applied to drug treatment purposes.

EP 4 617 281 A1

## Description

### Technical Field

The present invention belongs to the field of biopharmaceutical technology, and particularly relates to a site-specifically PEGylated heteromeric protein or polypeptide and a preparation method and use of the same.

### Background Art

Since the research team led by Professor Frank F. Davis of Rutgers University found in 1977 that proteins can prolong their half-life and reduce immunogenicity by covalently coupling long-chain polyethylene glycol (Abuchowski et al. 1977a, 1977b), chemical modification with polyethylene glycol (PEGylation) has been widely used in a large number of bioactive drug molecules and has gradually become one of the most important ways to deliver long-acting preparations of protein and polypeptide drugs. After years of development, the technology of drug PEGylation entered the stage of industrialization and implementation in the 1980s. In 1990, the FDA approved the world's first PEGylated drug (PEGylated adenosine deaminase for the treatment of severe combined immunodeficiency) developed by Enzon, USA, which not only delayed the half-life of the drug, but also greatly reduced the drug toxicity and improved the quality of life of patients. In the following decades, the PEGylation technology has developed rapidly. Scientists have successfully developed protein drugs such as PEGylated adenylate deaminase (PEG-ADA), asparaginase (PEG-L-asparaginase), interferon α-2b (peginterferon α-2b), interferon α-2a (peginterferon α-2a), recombinant human granulocyte colony stimulating factor, as well as PEGylated camptothecin (PEG-CPT) and liposomal doxorubicin.

However, due to technical limitations, many of the early PEGylated drugs used random modification and multiple modifications. This type of modification often results in a mixture of drugs with non-uniform structures. The batch-to-batch differences in drug activity are difficult to control, and there is also great uncertainty in safety. With the development of drug PEGylation technology, there is a tendency to obtain drugs with definite structures by site-specific modification with a single PEG. Currently, commercially available PEGylated drugs basically employ site-specific modification of an N-terminal α-amino group of a protein with a polyethylene glycol aldehyde derivative or site-specific modification of thiol groups of a cysteine side chain of a protein with a polyethylene glycol maleimide derivative or vinyl sulfone. However, this site-specific modification only has the effect of site-specific single modification on a single-chain protein or a protein that contains a single free cysteine thiol group, and when used to modify a dimeric protein or a protein that contains multiple free cysteine thiol groups, it is no longer a site-specific single modification.

### Summary of the Invention

An object of the present invention is to provide a method for preparing a site-specifically PEGylated heteromeric protein or polypeptide.

Another object of the present invention is to provide a site-specifically PEGylated heteromeric protein or polypeptide.

Yet another object of the present invention is to provide related applications of the site-specifically PEGylated heteromeric protein or polypeptide.

The present invention provides a novel method for site-specific PEGylation of a heteromeric protein or polypeptide. By mutating a heteromeric protein or polypeptide composition, blocking the N-terminal α-amino group not intended to be modified with polyethylene glycol (PEG), while retaining only the N-terminal α-amino group intended to be modified with PEG, and then reacting an aldehyde derivative or ketone derivative of polyethylene glycol with the retained N-terminal α-amino group, site-specific modification of the heteromeric protein or polypeptide composition is achieved by this method.

Specifically, in one aspect, the present invention provides a method for site-specific PEGylation of a protein or polypeptide, the method including:

a) Providing a protein or polypeptide to be modified, the protein or polypeptide to be modified including a target N-terminal α-amino group and a non-target N-terminal α-amino group, and mutating and blocking the non-target N-terminal α-amino group; thereafter

b) Subjecting the target N-terminal α-amino group to a site-specific modification reaction with an aldehyde or ketone derivative of polyethylene glycol to generate a site-specifically PEGylated protein or polypeptide.

In the present invention, the "target N-terminal α-amino group" refers to the N-terminal α-amino group intended to be modified with polyethylene glycol, and the "non-target N-terminal α-amino group" refers to the N-terminal α-amino group not intended to be modified with polyethylene glycol.

Site-specific PEGylation of heteromeric proteins or polypeptides using the method of the present invention has a defined and controllable modification position, can bring a clearly extended half-life and biased composition activity to the modified product, and can be better applied to drug treatment purposes.

According to some specific embodiments of the present invention, in the method for site-specific PEGylation of a protein or polypeptide of the present invention, the protein or polypeptide is a heteromeric protein or polypeptide.

According to some specific embodiments of the present invention, in the method for site-specific PEGylation of a protein or polypeptide of the present invention, mutating and blocking the non-target N-terminal α-amino group includes:

constructing a gene encoding an amino acid sequence of the mutated and blocked protein or polypeptide into a vector, and then transforming the vector into a host bacterium and extracting an endotoxin-free plasmid,
thereafter transfecting the plasmid into a host cell to express a target protein,
and finally harvesting a target product which is the protein or polypeptide whose non-target N-terminal α-amino group is mutated and blocked.

According to some specific embodiments of the present invention, in the method for site-specific PEGylation of a protein or polypeptide of the present invention, the host cell is preferably a 293F cell.

According to some specific embodiments of the present invention, in the method for site-specific PEGylation of a protein or polypeptide of the present invention, the protein or polypeptide is a heteromeric protein or polypeptide including two to five N-terminal α-amino groups.

According to some specific embodiments of the present invention, in the method for site-specific PEGylation of a protein or polypeptide of the present invention, the heteromeric protein or polypeptide includes one target N-terminal α-amino group and one to two non-target N-terminal α-amino groups.

According to some specific embodiments of the present invention, in the method for site-specific PEGylation of a protein or polypeptide of the present invention, the heteromeric protein or polypeptide is a short half-life protein.

According to some specific embodiments of the present invention, in the method for site-specific PEGylation of a protein or polypeptide of the present invention, the short half-life protein is a two-subunit heteromeric protein or polypeptide .

According to some specific embodiments of the present invention, in the method for site-specific PEGylation of a protein or polypeptide of the present invention, the two- subunit heteromeric protein or polypeptide is selected from one or more of antibodys, cytokines, cytokine receptors, cytokine fusion proteins, hormones, hemoglobins, and G protein-coupled receptors.

According to some specific embodiments of the present invention, in the method for site-specific PEGylation of a protein or polypeptide of the present invention, the cytokine is selected from one or more of interleukins, interferons, lymphotoxins, growth factors, and chemokines.

According to some specific embodiments of the present invention, in the method for site-specific PEGylation of a protein or polypeptide of the present invention, the antibody is selected from a monoclonal antibody, a polyclonal antibody, and an antibody fragment. More specifically, the antibody fragment preferably includes a Fab fragment of the antibody.

According to some specific embodiments of the present invention, in the method for site-specific PEGylation of a protein or polypeptide of the present invention, preferably, the hormone is selected from one or more of follicle-stimulating hormone, luteinizing hormone, luteinizing hormone-releasing hormone, thyroid-stimulating hormone, and chorionic gonadotropin.

According to some specific embodiments of the present invention, in the method for site-specific PEGylation of a protein or polypeptide of the present invention, the interleukin is one or more of interleukin-6, interleukin-12, interleukin-23, interleukin-27 and interleukin-35.

According to some specific embodiments of the present invention, in the method for site-specific PEGylation of a protein or polypeptide of the present invention, the interleukin-12 includes human interleukin-12 and mouse interleukin-12.

According to some specific embodiments of the present invention, in the method for site-specific PEGylation of a protein or polypeptide of the present invention, the interleukin-12 includes two subunits, p35 and p40.

According to some specific embodiments of the present invention, in the method for site-specific PEGylation of a protein or polypeptide of the present invention, the mutating and blocking the non-target N-terminal α-amino group includes:
mutating a first amino acid of a non-target N-terminal of the protein or polypeptide to be modified to one or more of proline, glutamine, glutamic acid, asparagine, aspartic acid, or unnatural amino acid by adding, deleting, or substituting one or more amino acid residues.

According to some specific embodiments of the present invention, in the method for site-specific PEGylation of a protein or polypeptide of the present invention, the mutated amino acid may be proline or glutamine.

According to some specific embodiments of the present invention, in the method for site-specific PEGylation of a protein or polypeptide of the present invention, the aldehyde or ketone derivative of polyethylene glycol is selected from

one or more of a polyethylene glycol acetaldehyde derivative, a polyethylene glycol propionaldehyde derivative, and a polyethylene glycol butyraldehyde derivative. Further, the polyethylene glycol propionaldehyde derivative is monomethoxy polyethylene glycol propionaldehyde and/or two-arm polyethylene glycol propionaldehyde.

In another aspect, the present invention also provides a site-specifically PEGylated protein or polypeptide, in which a non-PEGylated N-terminal in the amino acid sequence of the protein or polypeptide is mutated and blocked with respect to a wild-type protein or polypeptide.

According to some specific embodiments of the present invention, the site-specifically PEGylated protein or polypeptide of the present invention is prepared according to the method for site-specific PEGylation of a protein or polypeptide according to the present invention.

According to some specific embodiments of the present invention, the site-specifically PEGylated protein or polypeptide of the present invention has a molecular weight of 5 kD to 40 kD, preferably 10 kD to 30 kD, and more preferably about 20 kD.

According to some specific embodiments of the present invention, in the site-specifically PEGylated protein or polypeptide of the present invention, one end of a polyethylene glycol structure is connected to a target N-terminal of the protein or polypeptide for modification, and the other end of the polyethylene glycol structure is modified with an immunoconjugate or a targeting fragment.

According to some specific embodiments of the present invention, in the site-specifically PEGylated protein or polypeptide of the present invention, the immunoconjugate or targeting fragment is to further extend the half-life of the site-specifically PEGylated protein or enhance the targeting thereof. Immunoconjugates or targeting fragments with such function can be selected with reference to the related art in the field. For example, the immunoconjugate or targeting fragment can be selected from albumin, albumin fusion protein, antibodys, cytokines, cytokine receptors, cytokine fusion proteins, hormones, hemoglobins, G protein-coupled receptors, PAS, HES, CTP of a β subunit of human chorionic gonadotropin, PEG, and XTEN. Preferably, the cytokine is selected from interleukin, interferon, lymphotoxin, a growth factor, and a chemokine. Preferably, the antibody is selected from monoclonal antibodys, a polyclonal antibodys, and antibody fragments. Preferably, the antibody fragment is an Fc fragment of an IgG2 or IgG4 type antibody. Preferably, the antibody fragment is a tumor-targeting antibody fragment. Preferably, the hormone is selected from follicle-stimulating hormone, luteinizing hormone, luteinizing hormone-releasing hormone, thyroid-stimulating hormone, and chorionic gonadotropin. Preferably, interleukins include interleukin-12, interleukin-2, interleukin-15, interleukin-23, interleukin-27, interleukin-35 or the like. Preferably, the immunoconjugate or targeting fragment has a different structure from that of the protein or polypeptide at the other end of the polyethylene glycol structure.

According to some specific embodiments of the present invention, in the site-specifically PEGylated protein or polypeptide of the present invention, an amino acid sequence of the protein or polypeptide includes one or more of the following amino acid sequences: SEQ ID NO. 3, SEQ ID NO. 4, SEQ ID NO. 5; SEQ ID NO. 6, SEQ ID NO. 7, SEQ ID NO. 8; SEQ ID NO. 11, SEQ ID NO. 12, SEQ ID NO. 13; SEQ ID NO. 14, 15, SEQ ID NO. 16; SEQ ID NO. 26, SEQ ID NO. 27, SEQ ID NO. 28; and SEQ ID NO. 33, SEQ ID NO. 34, SEQ ID NO. 35.

According to some specific embodiments of the present invention, in the site-specifically PEGylated protein or polypeptide of the present invention, the structure of the protein or polypeptide is selected from one or more of the following proteins or polypeptides:

mutant human interleukin-12 with a p35 subunit having an amino acid sequence set forth in any one of SEQ ID NOs. 3 to 5,
mutant human interleukin-12 with a p40 subunit having an amino acid sequence set forth in any one of SEQ ID NOs. 6 to 8,
mutant mouse interleukin-12 with a p35 subunit having an amino acid sequence set forth in any one of SEQ ID NOs. 11 to 13,
mutant mouse interleukin-12 with a p40 subunit having an amino acid sequence set forth in any one of SEQ ID NOs. 14 to 16,
mutant human luteinizing hormone with an α subunit having an amino acid sequence set forth in any one of SEQ ID NOs. 26 to 28 and a β subunit having an amino acid sequence set forth in SEQ ID NO. 18,
mutant mouse luteinizing hormone with an α subunit having an amino acid sequence set forth in any one of SEQ ID NOs. 33 to 35 and a β subunit having an amino acid sequence set forth in SEQ ID NO. 30,
mutant human thyroid-stimulating hormone with an α subunit having an amino acid sequence set forth in any one of SEQ ID NOs. 26 to 28 and a β subunit having an amino acid sequence set forth in SEQ ID NO. 19,
mutant mouse thyroid-stimulating hormone with an α subunit having an amino acid sequence set forth in any one of SEQ ID NOs. 33 to 35 and a β subunit having an amino acid sequence set forth in SEQ ID NO. 31,
mutant human follicle-stimulating hormone with an α subunit having an amino acid sequence set forth in any one of SEQ ID NOs. 26 to 28 and a β subunit having an amino acid sequence set forth in SEQ ID NO. 20,
mutant mouse follicle-stimulating hormone with an α subunit having an amino acid sequence set forth in any one of

SEQ ID NOs. 33 to 35 and a β subunit having an amino acid sequence set forth in SEQ ID NO. 32, and
mutant human chorionic gonadotropin with an α subunit having an amino acid sequence set forth in any one of SEQ ID NOs. 26 to 28 and a β subunit having an amino acid sequence set forth in any one of SEQ ID NOs. 21 to 25.

In another aspect, the present invention also provides a medicament including: the site-specifically PEGylated protein or polypeptide according to the present invention; and a pharmaceutically acceptable excipient.

According to some specific embodiments of the present invention, a dosage form of the medicament of the present invention includes a liquid preparation and/or a lyophilized preparation.

According to some specific embodiments of the present invention, an administration route of the medicament of the present invention includes injection, oral administration, topical application, or combined administration via multiple routes.

In another aspect, the present invention also provides a use of the site-specifically PEGylated protein or polypeptide or the medicament in the manufacture of an anti-tumor medicament.

According to some specific embodiments of the present invention, the anti-tumor medicament of the present invention can be administered to a patient alone or in combination with radiation therapy, chemotherapy, myeloablative therapy, CAR-T therapy, CAR-NK therapy, antibody targeted therapy, oncolytic virus therapy and/or gene vaccine therapy.

In another aspect, the present invention also provides a protein or polypeptide, which can be used as a precursor or intermediate for preparing the site-specifically PEGylated protein or polypeptide of the present invention. Specifically, and amino acid sequence of the protein or polypeptide includes one or more of the following:
SEQ ID NO. 3, SEQ ID NO. 4, SEQ ID NO. 5, SEQ ID NO. 6, SEQ ID NO. 7, SEQ ID NO. 8; SEQ ID NO. 11, SEQ ID NO. 12, SEQ ID NO. 13; SEQ ID NO. 14, SEQ ID NO. 15, SEQ ID NO. 16; SEQ ID NO. 26, SEQ ID NO. 27, SEQ ID NO. 28; and SEQ ID NO. 33, SEQ ID NO. 34, SEQ ID NO. 35.

According to some specific embodiments of the present invention, the protein or polypeptide of the present invention that can be used as a precursor or intermediate for preparing the site-specifically PEGylated protein or polypeptide of the present invention is selected from one or more of the following proteins or polypeptides:

mutant human interleukin-12 with a p35 subunit having an amino acid sequence set forth in any one of SEQ ID NOs. 3 to 5,
mutant human interleukin-12 with a p40 subunit having an amino acid sequence set forth in any one of SEQ ID NOs. 6 to 8,
mutant mouse interleukin-12 with a p35 subunit having an amino acid sequence set forth in any one of SEQ ID NOs. 11 to 13,
mutant mouse interleukin-12 with a p40 subunit having an amino acid sequence set forth in any one of SEQ ID NOs. 14 to 16,
mutant human luteinizing hormone with an α subunit having an amino acid sequence set forth in any one of SEQ ID NOs. 26 to 28 and a β subunit having an amino acid sequence set forth in SEQ ID NO. 18,
mutant mouse luteinizing hormone with an α subunit having an amino acid sequence set forth in any one of SEQ ID NOs. 33 to 35 and a β subunit having an amino acid sequence set forth in SEQ ID NO. 30,
mutant human thyroid-stimulating hormone with an α subunit having an amino acid sequence set forth in any one of SEQ ID NOs. 26 to 28 and a β subunit having an amino acid sequence set forth in SEQ ID NO. 19,
mutant mouse thyroid-stimulating hormone with an α subunit having an amino acid sequence set forth in any one of SEQ ID NOs. 33 to 35 and a β subunit having an amino acid sequence set forth in SEQ ID NO. 31,
mutant human follicle-stimulating hormone with an α subunit having an amino acid sequence set forth in any one of SEQ ID NOs. 26 to 28 and a β subunit having an amino acid sequence set forth in SEQ ID NO. 20,
mutant mouse follicle-stimulating hormone with an α subunit having an amino acid sequence set forth in any one of SEQ ID NOs. 33 to 35 and a β subunit having an amino acid sequence set forth in SEQ ID NO. 32, and
mutant human chorionic gonadotropin with an α subunit having an amino acid sequence set forth in any one of SEQ ID NOs. 26 to 28 and a β subunit having an amino acid sequence set forth in any one of SEQ ID NOs. 21 to 25.

In another aspect, the present invention also provides a nucleic acid molecule encoding the protein or polypeptide of the present invention.

According to some specific embodiments of the present invention, the nucleic acid molecule of the present invention includes one or more of the following nucleotide sequences:
SEQ ID NO. 38, SEQ ID NO. 39, SEQ ID NO. 40; SEQ ID NO. 41, SEQ ID NO.42, SEQ ID NO. 43; SEQ ID NO. 46, SEQ ID NO. 47, SEQ ID NO.48; SEQ ID NO. 49, SEQ ID NO. 50, SEQ ID NO. 51; SEQ ID NO. 61, SEQ ID NO. 62, SEQ ID NO. 63; and SEQ ID NO. 68, SEQ ID NO. 69, SEQ ID NO.70.

In some specific embodiments of the present invention, blocking an N-terminal α-amino group of another subunit of the heteromeric protein or polypeptide composition by gene mutation includes mutating the N-terminal amino acid of the

blocked subunit by addition, deletion or substitution to proline (Pro, P), which forms a ring by itself to block the N-terminal α-amino group, or mutating the N-terminal amino acid of the blocked subunit to glutamine, glutamic acid, asparagine, or aspartic acid and reacting the side chain carboxyl group with the N-terminal α-amino group to form a ring by means of a glutamine cyclase or the like, or blocking the N-terminal α-amino group or weakening the reaction probability of the N-terminal α-amino group with the aldehyde derivative or ketone derivative of polyethylene glycol by inserting an unnatural amino acid. Further, by reacting the aldehyde derivative or ketone derivative of polyethylene glycol with the heteromeric protein or polypeptide composition, and controlling the reaction conditions to keep the reaction pH acidic, the aldehyde derivative or ketone derivative of polyethylene glycol only reacts with the retained N-terminal α-amino group but not with the ε-amino group of the amino acid side chain.

In some specific embodiments of the present invention, the N-terminal α-amino group of a redundant subunit is blocked by gene mutation technology, so as to achieve the site-specific single modification of the N-terminal α-amino group of the target subunit of the heteromeric protein or polypeptide composition by the aldehyde derivative or ketone derivative of polyethylene glycol. The obtained product has a defined and consistent structure, and thus has definite long-term effectiveness, safety and effectiveness when it is used in drug treatment. At the same time, by blocking different subunits, the position modified with polyethylene glycol can be changed. Changing the position modified with polyethylene glycol brings steric hindrance, which changes the binding ability of the protein or polypeptide composition with a receptor protein, affects the downstream signaling pathways, brings about changes in the drug treatment efficacy, and achieves better drug treatment objectives. When using double-end modified polyethylene glycol derivatives, antibody fragments can be introduced to enhance the transport and targeting ability of drugs, or other drugs can be introduced to achieve the effect of combined treatment.

The amino acid and nucleotide sequences mentioned in the present invention are as follows:

SEQ ID NO. 1: amino acid sequence of wild-type human IL-12 P35 subunit:

RNLPVATPDPGMFPCLHHSQNLLRAVSNMLQKARQTLEFYPCTSEEIDHEDITKD
KTSTVEACLPLELTKNESCLNSRETSFITNGSCLASRKTSFMMALCLSSIYEDLKMYQ
VEFKTMNAKLLMDPKRQIFLDQNMLAVIDELMQALNFNSETVPQKSSLEEPDFYKTK
IKLCILLHAFRIRAVTIDRVMSYLNAS

SEQ ID NO. 2: amino acid sequence of wild-type human IL-12 P40 subunit:

IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCE
AKNYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLKN
SRQVEVSWEYPDTWSTPHSYFSLTFCVQVQGKSKREKKDRVFTDKTSATVICRKNASI
SVRAQDRYYSSSWSEWASVPCS

SEQ ID NO. 3: amino acid sequence of mutant human IL-12 P35 subunit:

PNLPVATPDPGMFPCLHHSQNLLRAVSNMLQKARQTLEFYPCTSEEIDHEDITKD
KTSTVEACLPLELTKNESCLNSRETSFITNGSCLASRKTSFMMALCLSSIYEDLKMYQ
VEFKTMNAKLLMDPKRQIFLDQNMLAVIDELMQALNFNSETVPQKSSLEEPDFYKTK
IKLCILLHAFRIRAVTIDRVMSYLNAS

SEQ ID NO. 4: amino acid sequence of mutant human IL-12 P35 subunit:

QNLPVATPDPGMFPCLHHSQNLLRAVSNMLQKARQTLEFYPCTSEEIDHEDITKD KTSTVEACLPLELTKNESCLNSRETSFITNGSCLASRKTSFMMALCLSSIYEDLKMYQ VEFKTMNAKLLMDPKRQIFLDQNMLAVIDELMQALNFNSETVPQKSSLEEPDFYKTK IKLCILLHAFRIRAVTIDRVMSYLNAS

SEQ ID NO. 5: amino acid sequence of mutant human IL-12 P35 subunit:

NNLPVATPDPGMFPCLHHSQNLLRAVSNMLQKARQTLEFYPCTSEEIDHEDITKD KTSTVEACLPLELTKNESCLNSRETSFITNGSCLASRKTSFMMALCLSSIYEDLKMYQ VEFKTMNAKLLMDPKRQIFLDQNMLAVIDELMQALNFNSETVPQKSSLEEPDFYKTK IKLCILLHAFRIRAVTIDRVMSYLNAS

SEQ ID NO. 6: amino acid sequence of mutant human IL-12 P40 subunit:

PWELKKDVYVVELDWYPDAPGEMVVLTCDTPEEDGITWTLDQSSEVLGSGKTL TIQVKEFGDAGQYTCHKGGEVLSHSLLLLHKKEDGIWSTDILKDQKEPKNKTFLRCE AKNYSGRFTCWWLTTISTDLTFSVKSSRGSSDPQGVTCGAATLSAERVRGDNKEYEY SVECQEDSACPAAEESLPIEVMVDAVHKLKYENYTSSFFIRDIIKPDPPKNLQLKPLKN SRQVEVSWEYPDTWSTPHSYFSLTFCVQVQGKSKREKKDRVFTDKTSATVICRKNASI SVRAQDRYYSSSWSEWASVPCS

SEQ ID NO. 7: amino acid sequence of mutant human IL-12 P40 subunit:

QWELKKDVYVVELDWYPDAPGEMVVLTCDTPEEDGITWTLDQSSEVLGSGKTL TIQVKEFGDAGQYTCHKGGEVLSHSLLLLHKKEDGIWSTDILKDQKEPKNKTFLRCE AKNYSGRFTCWWLTTISTDLTFSVKSSRGSSDPQGVTCGAATLSAERVRGDNKEYEY SVECQEDSACPAAEESLPIEVMVDAVHKLKYENYTSSFFIRDIIKPDPPKNLQLKPLKN SRQVEVSWEYPDTWSTPHSYFSLTFCVQVQGKSKREKKDRVFTDKTSATVICRKNASI SVRAQDRYYSSSWSEWASVPCS

SEQ ID NO. 8: amino acid sequence of mutant human IL-12 P40 subunit:

NWELKKDVYVVELDWYPDAPGEMVVLTCDTPEEDGITWTLDQSSEVLGSGKTL TIQVKEFGDAGQYTCHKGGEVLSHSLLLLHKKEDGIWSTDILKDQKEPKNKTFLRCE AKNYSGRFTCWWLTTISTDLTFSVKSSRGSSDPQGVTCGAATLSAERVRGDNKEYEY SVECQEDSACPAAEESLPIEVMVDAVHKLKYENYTSSFFIRDIIKPDPPKNLQLKPLKN SRQVEVSWEYPDTWSTPHSYFSLTFCVQVQGKSKREKKDRVFTDKTSATVICRKNASI SVRAQDRYYSSSWSEWASVPCS

SEQ ID NO. 9: amino acid sequence of wild-type mouse IL-12 P35 subunit:

RVIPVSGPARCLSQSRNLLKTTDDMVKTAREKLKHYSCTAEDIDHEDITRDQTST LKTCLPLELHKNESCLATRETSSTTRGSCLPPQKTSLMMTLCLGSIYEDLKMYQTEFQ AINAALQNHNHQQIILDKGMLVAIDELMQSLNHNGETLRQKPPVGEADPYRVKMKLC ILLHAFSTRVVTINRVMGYLSSA

SEQ ID NO. 10: amino acid sequence of wild-type mouse IL-12 P40 subunit:

MWELEKDVYVVEVDWTPDAPGETVNLTCDTPEEDDITWTSDQRHGVIGSGKTL TITVKEFLDAGQYTCHKGGETLSHSHLLLHKKENGIWSTEILKNFKNKTFLKCEAPNY SGRFTCSWLVQRNMDLKFNIKSSSSSPDSRAVTCGMASLSAEKVTLDQRDYEKYSVS CQEDVTCPTAEETLPIELALEARQQNKYENYSTSFFIRDIIKPDPPKNLQMKPLKNSQV EVSWEYPDSWSTPHSYFSLKFFVRIQRKKEKMKETEEGCNQKGAFLVEKTSTEVQCK GGNVCVQAQDRYYNSSCSKWACVPCRVRS

SEQ ID NO. 11: amino acid sequence of mutant mouse IL-12 P35 subunit:

PVIPVSGPARCLSQSRNLLKTTDDMVKTAREKLKHYSCTAEDIDHEDITRDQTST LKTCLPLELHKNESCLATRETSSTTRGSCLPPQKTSLMMTLCLGSIYEDLKMYQTEFQ AINAALQNHNHQQIILDKGMLVAIDELMQSLNHNGETLRQKPPVGEADPYRVKMKLC ILLHAFSTRVVTINRVMGYLSS

SEQ ID NO. 12: amino acid sequence of mutant mouse IL-12 P35 subunit:

QVIPVSGPARCLSQSRNLLKTTDDMVKTAREKLKHYSCTAEDIDHEDITRDQTST LKTCLPLELHKNESCLATRETSSTTRGSCLPPQKTSLMMTLCLGSIYEDLKMYQTEFQ AINAALQNHNHQQIILDKGMLVAIDELMQSLNHNGETLRQKPPVGEADPYRVKMKLC ILLHAFSTRVVTINRVMGYLSS

SEQ ID NO. 13: amino acid sequence of mutant mouse IL-12 P35 subunit:

NVIPVSGPARCLSQSRNLLKTTDDMVKTAREKLKHYSCTAEDIDHEDITRDQTST LKTCLPLELHKNESCLATRETSSTTRGSCLPPQKTSLMMTLCLGSIYEDLKMYQTEFQ AINAALQNHNHQQIILDKGMLVAIDELMQSLNHNGETLRQKPPVGEADPYRVKMKLC ILLHAFSTRVVTINRVMGYLSS

SEQ ID NO. 14: amino acid sequence of mutant mouse IL-12 P40 subunit:

PWELEKDVYVVEVDWTPDAPGETVNLTCDTPEEDDITWTSDQRHGVIGSGKTLT ITVKEFLDAGQYTCHKGGETLSHSHLLLHKKENGIWSTEILKNFKNKTFLKCEAPNYS GRFTCSWLVQRNMDLKFNIKSSSSSPDSRAVTCGMASLSAEKVTLDQRDYEKYSVSC QEDVTCPTAEETLPIELALEARQQNKYENYSTSFFIRDIIKPDPPKNLQMKPLKNSQVE VSWEYPDSWSTPHSYFSLKFFVRIQRKKEKMKETEEGCNQKGAFLVEKTSTEVQCKG GNVCVQAQDRYYNSSCSKWACVPCRVRS

SEQ ID NO. 15: amino acid sequence of mutant mouse IL-12 P40 subunit:

QWELEKDVYVVEVDWTPDAPGETVNLTCDTPEEDDITWTSDQRHGVIGSGKTL TITVKEFLDAGQYTCHKGGETLSHSHLLLHKKENGIWSTEILKNFKNKTFLKCEAPNY SGRFTCSWLVQRNMDLKFNIKSSSSSPDSRAVTCGMASLSAEKVTLDQRDYEKYSVS CQEDVTCPTAEETLPIELALEARQQNKYENYSTSFFIRDIIKPDPPKNLQMKPLKNSQV EVSWEYPDSWSTPHSYFSLKFFVRIQRKKEKMKETEEGCNQKGAFLVEKTSTEVQCK GGNVCVQAQDRYYNSSCSKWACVPCRVRS

SEQ ID NO. 16: amino acid sequence of mutant mouse IL-12 P40 subunit:

NWELEKDVYVVEVDWTPDAPGETVNLTCDTPEEDDITWTSDQRHGVIGSGKTL TITVKEFLDAGQYTCHKGGETLSHSHLLLHKKENGIWSTEILKNFKNKTFLKCEAPNY SGRFTCSWLVQRNMDLKFNIKSSSSSPDSRAVTCGMASLSAEKVTLDQRDYEKYSVS CQEDVTCPTAEETLPIELALEARQQNKYENYSTSFFIRDIIKPDPPKNLQMKPLKNSQV EVSWEYPDSWSTPHSYFSLKFFVRIQRKKEKMKETEEGCNQKGAFLVEKTSTEVQCK GGNVCVQAQDRYYNSSCSKWACVPCRVRS

SEQ ID NO. 17: amino acid sequence of wild-type human glycoprotein hormone $\alpha$ subunit:

APDVQDCPECTLQENPFFSQPGAPILQCMGCCFSRAYPTPLRSKKTMLVQKNVTS ESTCCVAKSYNRVTVMGGFKVENHTACHCSTCYYHKS

SEQ ID NO. 18: amino acid sequence of wild-type human luteinizing hormone (hLH) $\beta$ subunit:

SREPLRPWCHPINAILAVEKEGCPVCITVNTTICAGYCPTMMRVLQAVLPPLPQVVCTYRDVRFESIRLPGCPRGVDPVVSFPVALSCRCGPCRRSTSDCGGPKDHPLTCDHPQLSGLLFL

SEQ ID NO. 19: amino acid sequence of wild-type human thyroid-stimulating hormone (hTSH) β subunit:

FCIPTEYTMHIERRECAYCLTINTTICAGYCMTRDINGKLFLPKYALSQDVCTYRDFIYRTVEIPGCPLHVAPYFSYPVALSCKCGKCNTDYSDCIHEAIKTNYCTKPQKSYLVGFSV

SEQ ID NO. 20: amino acid sequence of wild-type human follicle-stimulating hormone (hFSH) β subunit:

NSCELTNITIAIEKEECRFCISINTTWCAGYCYTRDLVYKDPARPKIQKTCTFKELVYETVRVPGCAHHADSLYTYPVATQCHCGKCDSDSTDCTVRGLGPSYCSFGEMKE

SEQ ID NO. 21: amino acid sequence of wild-type human chorionic gonadotropin (hCG) β1 subunit:

SKEPLRPRCRPINATLAVEKEGCPVCITVNTTICAGYCPTMTRVLQGVLPALPQVVCNYRDVRFESIRLPGCPRGVNPVVSYAVALSCQCALCRRSTTDCGGPKDHPLTCDDPRFQASSSSKAPPPSLPSPSRLPGPSDTPILPQ

SEQ ID NO. 22: amino acid sequence of wild-type human chorionic gonadotropin (hCG) β2 subunit:

SKEPLRPRCRPINATLAVEKEGCPVCITVNTTICAGYCPTMTRVLQGVLPALPQVVCNYRDVRFESIRLPGCPRGVNPVVSYAVALSCQCALCRRSTTDCGGPKDHPLTCDDPRFQDSSSSKAPPPSLPSPSRLPGPSDTPILPQ

SEQ ID NO. 23: amino acid sequence of wild-type human chorionic gonadotropin (hCG) β3 subunit:

SKEPLRPRCRPINATLAVEKEGCPVCITVNTTICAGYCPTMTRVLQGVLPALPQVVCNYRDVRFESIRLPGCPRGVNPVVSYAVALSCQCALCRRSTTDCGGPKDHPLTCDDPRFQDSSSSKAPPPSLPSPSRLPGPSDTPILPQ

SEQ ID NO. 24: amino acid sequence of wild-type human chorionic gonadotropin (hCG) β5 subunit:

SREMLRPRCRPINATLAVEKEGCPVCITVNTTICAGYCPTMTRVLQGVLPALPQVVCNYRDVRFESIRLPGCPRGVNPVVSYAVALSCQCALCRRSTTDCGGPKDHPLTCDDPRFQASSSSKAPPPSLPSPSRLPGPSDTPILPQ

SEQ ID NO. 25: amino acid sequence of wild-type human chorionic gonadotropin (hCG) β7 subunit:

SREMLRPRCRPINATLAVEKEGCPVCITVNTTICAGYCPTMTRVLQGVLPALPQV VCNYRDVRFESIRLPGCPRGVNPVVSYAVALSCQCALCRRSTTDCGGPKDHPLTCDDP RFQASSSSKAPPPSLPSPSRLPGPSDTPILPQ

SEQ ID NO. 26: amino acid sequence of mutant human glycoprotein hormone α subunit:

PPDVQDCPECTLQENPFFSQPGAPILQCMGCCFSRAYPTPLRSKKTMLVQKNVTS ESTCCVAKSYNRVTVMGGFKVENHTACHCSTCYYHKS

SEQ ID NO. 27: amino acid sequence of mutant human glycoprotein hormone α subunit:

QPDVQDCPECTLQENPFFSQPGAPILQCMGCCFSRAYPTPLRSKKTMLVQKNVTS ESTCCVAKSYNRVTVMGGFKVENHTACHCSTCYYHKS

SEQ ID NO. 28: amino acid sequence of mutant human glycoprotein hormone α subunit:

NPDVQDCPECTLQENPFFSQPGAPILQCMGCCFSRAYPTPLRSKKTMLVQKNVTS ESTCCVAKSYNRVTVMGGFKVENHTACHCSTCYYHKS

SEQ ID NO. 29: amino acid sequence of wild-type mouse glycoprotein hormone α subunit:

LPDGDFIIQGCPECKLKENKYFSKLGAPIYQCMGCCFSRAYPTPARSKKTMLVPK NITSEATCCVAKAFTKATVMGNARVENHTECHCSTCYYHKS

SEQ ID NO. 30: amino acid sequence of wild-type mouse luteinizing hormone (mLH) β subunit:

SRGPLRPLCRPVNATLAAENEFCPVCITFTTSICAGYCPSMVRVLPAALPPVPQPV CTYRELAFASVRLPGCPPGVDPIVSFPVALSCRCGPCRLSSSDCGGPRTQPMACDLPHL PGLLLL

SEQ ID NO. 31: amino acid sequence of wild-type mouse thyroid-stimulating hormone (mTSH) β subunit:

FCIPTEYTMYVDRRECAYCLTINTTICAGYCMTRDINGKLFLPKYALSQDVCTYR DFIYRTVEIPGCPHHVTPYFSFPVAVSCKCGKCNTDNSDCIHEAVRTNYCTKPQSFYLG GFSV

SEQ ID NO. 32: amino acid sequence of wild-type mouse follicle-stimulating hormone (mFSH) β subunit:

CELTNITISVEKEECRFCISINTTWCAGYCYTRDLVYKDPARPNTQKVCTFKELVY ETVRLPGCARHSDSLYTYPVATECHCGKCDSDSTDCTVRGLGPSYCSFSEMKE

SEQ ID NO. 33: amino acid sequence of mutant mouse glycoprotein hormone α subunit:

PPDGDFIIQGCPECKLKENKYFSKLGAPIYQCMGCCFSRAYPTPARSKKTMLVPKNITSEATCCVAKAFTKATVMGNARVENHTECHCSTCYYHKS

SEQ ID NO. 34: amino acid sequence of mutant mouse glycoprotein hormone α subunit:

QPDGDFIIQGCPECKLKENKYFSKLGAPIYQCMGCCFSRAYPTPARSKKTMLVPKNITSEATCCVAKAFTKATVMGNARVENHTECHCSTCYYHKS

SEQ ID NO. 35: amino acid sequence of mutant mouse glycoprotein hormone α subunit:

NPDGDFIIQGCPECKLKENKYFSKLGAPIYQCMGCCFSRAYPTPARSKKTMLVPKNITSEATCCVAKAFTKATVMGNARVENHTECHCSTCYYHKS

SEQ ID NO. 36: nucleotide sequence of wild-type human IL-12 P35 subunit:

cgcaacctgccggtggcgaccccggatccgggcatgtttccgtgcctgcatcatagccagaacctgctgcgcgcggtgagcaacatgctgcagaaagcgcgccagaccctggaattttatccgtgcaccagcgaagaaattgatcatgaagatattaccaaagataaaaccagcaccgtggaagcgtgcctgccgctggaactgaccaaaaacgaaagctgcctgaacagccgcgaaaccagctttattaccaacggcagctgcctggcgagccgcaaaaccagctttatgatggcgctgtgcctgagcagcatttatgaagatctgaaaatgtatcaggtggaatttaaaaccatgaacgcgaaactgctgatggatccgaaacgccagatttttctggatcagaacatgctggcggtgattgatgaactgatgcaggcgctgaactttaacagcgaaaccgtgccgcagaaaagcagcctggaagaaccggatttttataaaaccaaaattaaactgtgcattctgctgcatgcgtttcgcattcgcgcggtgaccattgatcgcgtgatgagctatctgaacgcgagc

SEQ ID NO. 37: nucleotide sequence of wild-type human IL-12 P40 subunit:

atttgggaactgaaaaaagatgtgtatgtggtggaactggattggtatccggatgcgccgggcgaaatggtggtgctgacctgcgataccccggaagaagatggcattacctggaccctggatcagagcagcgaagtgctgggcagcggcaaaaccctgaccattcaggtgaaagaatttggcgatgcgggccagtatacctgccataaaggcggcgaagtgctgagccatagcctgctgctgctgcataaaaaagaagatggcatttggagcaccgatattctgaaagatcagaaagaaccgaaaaacaaaacctttctgcgctgcgaagcgaaaaactatagcggccgctttacctgctggtggctgaccaccattagcaccgatctgacctttagcgtgaaaagcagccgcggcagcagcgatccgcagggcgtgacctgcggcgcggcgaccctgagcgcggaacgcgtgcgcggcgataacaaagaatatgaatatagcgtggaatgccaggaagatagcgcgtgcccggcggcggaagaaagcctgccgattgaagtgatggtggatgcggtgcataaactgaaatatgaaaactataccagcagctttttttattcgcgatattattaaaccggatccgccgaaaaacctgcagctgaaaccgctgaaaaacagccgccaggtggaagtgagctgggaatatccggatacctggagcaccccgcatagctattttagcctgaccttttgcgtgcaggtgcagggcaaaagcaaacgcgaaaaaaaagatcgcgtgtttaccgataaaaccagcgcgaccgtgatttgccgcaaaaacgcgagcattagcgtgcgcgcgcaggatcgctattatagcagcagctggagcgaatgggcgagcgtgccgtgcagc

SEQ ID NO. 38: nucleotide sequence of mutant human IL-12 P35 subunit:

ccgaacctgccggtggcgaccccggatccgggcatgtttccgtgcctgcatcatagccagaacctgctgcgcgcggtgagca acatgctgcagaaagcgcgccagaccctggaattttatccgtgcaccagcgaagaaattgatcatgaagatattaccaaagataaaacc agcaccgtggaagcgtgcctgccgctggaactgaccaaaaacgaaagctgcctgaacagccgcgaaaccagctttattaccaacgg cagctgcctggcgagccgcaaaaccagctttatgatggcgctgtgcctgagcagcatttatgaagatctgaaaatgtatcaggtggaatt taaaaccatgaacgcgaaactgctgatggatccgaaacgccagatttttctggatcagaacatgctggcggtgattgatgaactgatgca ggcgctgaactttaacagcgaaaccgtgccgcagaaaagcagcctggaagaaccggatttttataaaaccaaaattaaactgtgcattc tgctgcatgcgtttcgcattcgcgcggtgaccattgatcgcgtgatgagctatctgaacgcgagc

SEQ ID NO. 39: nucleotide sequence of mutant human IL-12 P35 subunit:

cagaacctgccggtggcgaccccggatccgggcatgtttccgtgcctgcatcatagccagaacctgctgcgcgcggtgagca acatgctgcagaaagcgcgccagaccctggaattttatccgtgcaccagcgaagaaattgatcatgaagatattaccaaagataaaacc agcaccgtggaagcgtgcctgccgctggaactgaccaaaaacgaaagctgcctgaacagccgcgaaaccagctttattaccaacgg cagctgcctggcgagccgcaaaaccagctttatgatggcgctgtgcctgagcagcatttatgaagatctgaaaatgtatcaggtggaatt taaaaccatgaacgcgaaactgctgatggatccgaaacgccagatttttctggatcagaacatgctggcggtgattgatgaactgatgca ggcgctgaactttaacagcgaaaccgtgccgcagaaaagcagcctggaagaaccggatttttataaaaccaaaattaaactgtgcattc tgctgcatgcgtttcgcattcgcgcggtgaccattgatcgcgtgatgagctatctgaacgcgagc

SEQ ID NO. 40: nucleotide sequence of mutant human IL-12 P35 subunit:

aacaacctgccggtggcgaccccggatccgggcatgtttccgtgcctgcatcatagccagaacctgctgcgcgcggtgagca acatgctgcagaaagcgcgccagaccctggaattttatccgtgcaccagcgaagaaattgatcatgaagatattaccaaagataaaacc agcaccgtggaagcgtgcctgccgctggaactgaccaaaaacgaaagctgcctgaacagccgcgaaaccagctttattaccaacgg cagctgcctggcgagccgcaaaaccagctttatgatggcgctgtgcctgagcagcatttatgaagatctgaaaatgtatcaggtggaatt taaaaccatgaacgcgaaactgctgatggatccgaaacgccagatttttctggatcagaacatgctggcggtgattgatgaactgatgca ggcgctgaactttaacagcgaaaccgtgccgcagaaaagcagcctggaagaaccggatttttataaaaccaaaattaaactgtgcattc tgctgcatgcgtttcgcattcgcgcggtgaccattgatcgcgtgatgagctatctgaacgcgagc

SEQ ID NO. 41: nucleotide sequence of mutant human IL-12 P40 subunit:

ccgtgggaactgaaaaaagatgtgtatgtggtggaactggattggtatccggatgcgccgggcgaaatggtggtgctgacctg cgataccccggaagaagatggcattacctggaccctggatcagagcagcgaagtgctgggcagcggcaaaaccctgaccattcagg tgaaagaatttggcgatgcgggccagtatacctgccataaaggcggcgaagtgctgagccatagcctgctgctgctgcataaaaaaga agatggcatttggagcaccgatattctgaaagatcagaaagaaccgaaaaacaaaacctttctgcgctgcgaagcgaaaaactatagc ggccgctttacctgctggtggctgaccaccattagcaccgatctgacctttagcgtgaaaagcagccgcggcagcagcgatccgcag ggcgtgacctgcggcgcggcgaccctgagcgcggaacgcgtgcgcggcgataacaaagaatatgaatatagcgtggaatgccagg aagatagcgcgtgcccggcggcggaagaaagcctgccgattgaagtgatggtggatgcggtgcataaactgaaatatgaaaactata ccagcagctttttttattcgcgatattattaaaccggatccgccgaaaaacctgcagctgaaaccgctgaaaaacagccgccaggtggaa gtgagctgggaatatccggatacctggagcaccccgcatagctattttagcctgaccttttgcgtgcaggtgcagggcaaaagcaaac gcgaaaaaaaagatcgcgtgtttaccgataaaaccagcgcgaccgtgatttgccgcaaaaacgcgagcattagcgtgcgcgcgcag gatcgctattatagcagcagctggagcgaatgggcgagcgtgccgtgcagc

SEQ ID NO. 42: nucleotide sequence of mutant human IL-12 P40 subunit:

cagtgggaactgaaaaaagatgtgtatgtggtggaactggattggtatccggatgcgccgggcgaaatggtggtgctgacctg cgataccccggaagaagatggcattacctggaccctggatcagagcagcgaagtgctgggcagcggcaaaaccctgaccattcagg tgaaagaatttggcgatgcgggccagtatacctgccataaaggcggcgaagtgctgagccatagcctgctgctgctgcataaaaaaga agatggcatttggagcaccgatattctgaaagatcagaaagaaccgaaaaacaaaacctttctgcgctgcgaagcgaaaaactatagc ggccgctttacctgctggtggctgaccaccattagcaccgatctgacctttagcgtgaaaagcagccgcggcagcagcgatccgcag ggcgtgacctgcggcgcggcgaccctgagcgcggaacgcgtgcgcggcgataacaaagaatatgaatatagcgtggaatgccagg aagatagcgcgtgcccggcggcggaagaaagcctgccgattgaagtgatggtggatgcggtgcataaactgaaatatgaaaactata ccagcagctttttttattcgcgatattattaaaccggatccgccgaaaaacctgcagctgaaaccgctgaaaaacagccgccaggtggaa gtgagctgggaatatccggatacctggagcaccccgcatagctattttagcctgaccttttgcgtgcaggtgcagggcaaaagcaaac gcgaaaaaaaagatcgcgtgtttaccgataaaaccagcgcgaccgtgatttgccgcaaaaacgcgagcattagcgtgcgcgcgcag gatcgctattatagcagcagctggagcgaatgggcgagcgtgccgtgcagc

SEQ ID NO. 43: nucleotide sequence of mutant human IL-12 P40 subunit:

aactgggaactgaaaaaagatgtgtatgtggtggaactggattggtatccggatgcgccgggcgaaatggtggtgctgacctg cgataccccggaagaagatggcattacctggaccctggatcagagcagcgaagtgctgggcagcggcaaaaccctgaccattcagg

tgaaagaatttggcgatgcgggccagtatacctgccataaaggcggcgaagtgctgagccatagcctgctgctgctgcataaaaaagaagatggcatttggagcaccgatattctgaaagatcagaaagaaccgaaaaacaaaacctttctgcgctgcgaagcgaaaaactatagcggccgctttacctgctggtggctgaccaccattagcaccgatctgacctttagcgtgaaaagcagccgcggcagcagcgatccgcagggcgtgacctgcggcgcggcgaccctgagcgcggaacgcgtgcgcggcgataacaaagaatatgaatatagcgtggaatgccaggaagatagcgcgtgcccggcggcggaagaaagcctgccgattgaagtgatggtggatgcggtgcataaactgaaatatgaaaactataccagcagctttttttattcgcgatattattaaaccggatccgccgaaaaacctgcagctgaaaccgctgaaaaacagccgccaggtggaagtgagctgggaatatccggatacctggagcaccccgcatagctattttagcctgacctttttgcgtgcaggtgcagggcaaaagcaaacgcgaaaaaaaagatcgcgtgtttaccgataaaaccagcgcgaccgtgatttgccgcaaaaacgcgagcattagcgtgcgcgcgcaggatcgctattatagcagcagctggagcgaatgggcgagcgtgccgtgcagc

SEQ ID NO. 44: nucleotide sequence of wild-type mouse IL-12 P35 subunit:

cgcgtgattccggtgagcggcccggcgcgctgcctgagccagagccgcaacctgctgaaaaccaccgatgatatggtgaaaaccgcgcgcgaaaaactgaaacattatagctgcaccgcggaagatattgatcatgaagatattacccgcgatcagaccagcaccctgaaaacctgcctgccgctggaactgcataaaaacgaaagctgcctggcgacccgcgaaaccagcagcaccacccgcggcagctgcctgccgccgcagaaaaccagcctgatgatgaccctgtgcctgggcagcatttatgaagatctgaaaatgtatcagaccgaatttcaggcgattaacgcggcgctgcagaaccataaccatcagcagattattctggataaaggcatgctggtggcgattgatgaactgatgcagagcctgaaccataacggcgaaaccctgcgccagaaaccgccggtgggcgaagcggatccgtatcgcgtgaaaatgaaactgtgcattctgctgcatgcgtttagcacccgcgtggtgaccattaaccgcgtgatgggctatctgagcagcgcg

SEQ ID NO. 45: nucleotide sequence of wild-type mouse IL-12 P40 subunit:

atgtgggaactggaaaaagatgtgtatgtggtggaagtggattggaccccggatgcgccgggcgaaaccgtgaacctgacctgcgataccccggaagaagatgatattacctggaccagcgatcagcgccatggcgtgattggcagcggcaaaaccctgaccattaccgtgaaagaatttctggatgcgggccagtatacctgccataaaggcggcgaaaccctgagccatagccatctgctgctgcataaaaaagaaaacggcatttggagcaccgaaattctgaaaaactttaaaaacaaaacctttctgaaatgcgaagcgccgaactatagcggccgctttacctgcagctggctggtgcagcgcaacatggatctgaaatttaacattaaaagcagcagcagcagcccggatagccgcgcggtgacctgcggcatggcgagcctgagcgcggaaaaagtgaccctggatcagcgcgattatgaaaaatatagcgtgagctgccaggaagatgtgacctgcccgaccgcggaagaaaccctgccgattgaactggcgctggaagcgcgccagcagaacaaatatgaaaactatagcaccagctttttttattcgcgatattattaaaccggatccgccgaaaaacctgcagatgaaaccgctgaaaaacagccaggtggaagtgagctgggaatatccggatagctggagcaccccgcatagctattttagcctgaaatttttttgtgcgcattcagcgcaaaaaagaaaaaatgaaagaaaccgaagaaggctgcaaccagaaaggcgcgtttctggtggaaaaaaccagcaccgaagtgcagtgcaaaggcggcaacgtgtgcgtgcaggcgcaggatcgctattataacagcagctgcagcaaatgggcgtgcgtgccgtgccgcgtgcgcagc

SEQ ID NO. 46: nucleotide sequence of mutant mouse IL-12 P35 subunit:

ccggtgattccggtgagcggcccggcgcgctgcctgagccagagccgcaacctgctgaaaaccaccgatgatatggtgaaaaccgcgcgcgaaaaactgaaacattatagctgcaccgcggaagatattgatcatgaagatattacccgcgatcagaccagcaccctgaaaacctgcctgccgctggaactgcataaaaacgaaagctgcctggcgacccgcgaaaccagcagcaccacccgcggcagctgcctgccgccgcagaaaaccagcctgatgatgaccctgtgcctgggcagcatttatgaagatctgaaaatgtatcagaccgaatttcaggcgattaacgcggcgctgcagaaccataaccatcagcagattattctggataaaggcatgctggtggcgattgatgaactgatgcagagcctgaaccataacggcgaaaccctgcgccagaaaccgccggtgggcgaagcggatccgtatcgcgtgaaaatgaaactgtgcattctgctgcatgcgtttagcacccgcgtggtgaccattaaccgcgtgatgggctatctgagcagc

SEQ ID NO. 47: nucleotide sequence of mutant mouse IL-12 P35 subunit:

caggtgattccggtgagcggcccggcgcgctgcctgagccagagccgcaacctgctgaaaaccaccgatgatatggtgaaaaccgcgcgcgaaaaactgaaacattatagctgcaccgcggaagatattgatcatgaagatattacccgcgatcagaccagcaccctgaaaacctgcctgccgctggaactgcataaaaacgaaagctgcctggcgacccgcgaaaccagcagcaccacccgcggcagctgcctgccgccgcagaaaaccagcctgatgatgaccctgtgcctgggcagcatttatgaagatctgaaaatgtatcagaccgaatttcaggcgattaacgcggcgctgcagaaccataaccatcagcagattattctggataaaggcatgctggtggcgattgatgaactgatgcagagcctgaaccataacggcgaaaccctgcgccagaaaccgccggtgggcgaagcggatccgtatcgcgtgaaaatgaaactgtgcattctgctgcatgcgtttagcacccgcgtggtgaccattaaccgcgtgatgggctatctgagcagc

SEQ ID NO. 48: nucleotide sequence of mutant mouse IL-12 P35 subunit:

aacgtgattccggtgagcggcccggcgcgctgcctgagccagagccgcaacctgctgaaaaccaccgatgatatggtgaaaaccgcgcgcgaaaaactgaaacattatagctgcaccgcggaagatattgatcatgaagatattacccgcgatcagaccagcaccctgaaaacctgcctgccgctggaactgcataaaaacgaaagctgcctggcgacccgcgaaaccagcagcaccacccgcggcagctgcctgccgccgcagaaaaccagcctgatgatgaccctgtgcctgggcagcatttatgaagatctgaaaatgtatcagaccgaatttcaggcgattaacgcggcgctgcagaaccataaccatcagcagattattctggataaaggcatgctggtggcgattgatgaactgatgcagagcctgaaccataacggcgaaaccctgcgccagaaaccgccggtgggcgaagcggatccgtatcgcgtgaaaatgaaactgtgcattctgctgcatgcgtttagcacccgcgtggtgaccattaaccgcgtgatgggctatctgagcagc

SEQ ID NO. 49: nucleotide sequence of mutant mouse IL-12 P40 subunit:

ccgtgggaactggaaaaagatgtgtatgtggtggaagtggattggaccccggatgcgccgggcgaaaccgtgaacctgacctgcgataccccggaagaagatgatattacctggaccagcgatcagcgccatggcgtgattggcagcggcaaaaccctgaccattaccgtgaaagaatttctggatgcgggccagtatacctgccataaaggcggcgaaaccctgagccatagccatctgctgctgcataaaaaagaaaacggcatttggagcaccgaaattctgaaaaactttaaaaacaaaacctttctgaaatgcgaagcgccgaactatagcggccgctttac

ctgcagctggctggtgcagcgcaacatggatctgaaatttaacattaaaagcagcagcagcagcccggatagccgcgcggtgacctg cggcatggcgagcctgagcgcggaaaaagtgaccctggatcagcgcgattatgaaaaatatagcgtgagctgccaggaagatgtga cctgcccgaccgcggaagaaaccctgccgattgaactggcgctggaagcgcgccagcagaacaaatatgaaaactatagcaccagc tttttattcgcgatattattaaaccggatccgccgaaaaacctgcagatgaaaccgctgaaaaacagccaggtggaagtgagctggga atatccggatagctggagcaccccgcatagctattttagcctgaaattttttgtgcgcattcagcgcaaaaaagaaaaaatgaaagaaac cgaagaaggctgcaaccagaaaggcgcgtttctggtggaaaaaaccagcaccgaagtgcagtgcaaaggcggcaacgtgtgcgtg caggcgcaggatcgctattataacagcagctgcagcaaatgggcgtgcgtgccgtgccgcgtgcgcagc

SEQ ID NO. 50: nucleotide sequence of mutant mouse IL-12 P40 subunit:

cagtgggaactggaaaaagatgtgtatgtggtggaagtggattggaccccggatgcgccgggcgaaaccgtgaacctgacct gcgataccccggaagaagatgatattacctggaccagcgatcagcgccatggcgtgattggcagcggcaaaaccctgaccattaccg tgaaagaatttctggatgcgggccagtatacctgccataaaggcggcgaaaccctgagccatagccatctgctgctgcataaaaaaga aaacggcatttggagcaccgaaattctgaaaaactttaaaaacaaaacctttctgaaatgcgaagcgccgaactatagcggccgctttac ctgcagctggctggtgcagcgcaacatggatctgaaatttaacattaaaagcagcagcagcagcccggatagccgcgcggtgacctg cggcatggcgagcctgagcgcggaaaaagtgaccctggatcagcgcgattatgaaaaatatagcgtgagctgccaggaagatgtga cctgcccgaccgcggaagaaaccctgccgattgaactggcgctggaagcgcgccagcagaacaaatatgaaaactatagcaccagc tttttattcgcgatattattaaaccggatccgccgaaaaacctgcagatgaaaccgctgaaaaacagccaggtggaagtgagctggga atatccggatagctggagcaccccgcatagctattttagcctgaaattttttgtgcgcattcagcgcaaaaaagaaaaaatgaaagaaac cgaagaaggctgcaaccagaaaggcgcgtttctggtggaaaaaaccagcaccgaagtgcagtgcaaaggcggcaacgtgtgcgtg caggcgcaggatcgctattataacagcagctgcagcaaatgggcgtgcgtgccgtgccgcgtgcgcagc

SEQ ID NO. 51: nucleotide sequence of mutant mouse IL-12 P40 subunit:

aactgggaactggaaaaagatgtgtatgtggtggaagtggattggaccccggatgcgccgggcgaaaccgtgaacctgacct gcgataccccggaagaagatgatattacctggaccagcgatcagcgccatggcgtgattggcagcggcaaaaccctgaccattaccg tgaaagaatttctggatgcgggccagtatacctgccataaaggcggcgaaaccctgagccatagccatctgctgctgcataaaaaaga aaacggcatttggagcaccgaaattctgaaaaactttaaaaacaaaacctttctgaaatgcgaagcgccgaactatagcggccgctttac ctgcagctggctggtgcagcgcaacatggatctgaaatttaacattaaaagcagcagcagcagcccggatagccgcgcggtgacctg cggcatggcgagcctgagcgcggaaaaagtgaccctggatcagcgcgattatgaaaaatatagcgtgagctgccaggaagatgtga cctgcccgaccgcggaagaaaccctgccgattgaactggcgctggaagcgcgccagcagaacaaatatgaaaactatagcaccagc tttttattcgcgatattattaaaccggatccgccgaaaaacctgcagatgaaaccgctgaaaaacagccaggtggaagtgagctggga atatccggatagctggagcaccccgcatagctattttagcctgaaattttttgtgcgcattcagcgcaaaaaagaaaaaatgaaagaaac

cgaagaaggctgcaaccagaaaggcgcgtttctggtggaaaaaaccagcaccgaagtgcagtgcaaaggcggcaacgtgtgcgtgcaggcgcaggatcgctattataacagcagctgcagcaaatgggcgtgcgtgccgtgccgcgtgcgcagc

SEQ ID NO. 52: nucleotide sequence of wild-type human glycoprotein hormone α subunit:

gcgccggatgtgcaggattgcccggaatgcaccctgcaggaaaacccgtttttagccagccgggcgcgccgattctgcagtgcatgggctgctgctttagccgcgcgtatccgaccccgctgcgcagcaaaaaaaccatgctggtgcagaaaaacgtgaccagcgaaagcacctgctgcgtggcgaaaagctataaccgcgtgaccgtgatgggcggctttaaagtggaaaaccataccgcgtgccattgcagcacctgctattatcataaaagc

SEQ ID NO. 53: nucleotide sequence of wild-type human luteinizing hormone (hLH) β subunit:

agccgcgaaccgctgcgcccgtggtgccatccgattaacgcgattctggcggtggaaaaagaaggctgcccggtgtgcattaccgtgaacaccaccatttgcgcgggctattgcccgaccatgatgcgcgtgctgcaggcggtgctgccgccgctgccgcaggtggtgtgcacctatcgcgatgtgcgctttgaaagcattcgcctgccgggctgcccgcgcggcgtggatccggtggtgagctttccggtggcgctgagctgccgctgcggcccgtgccgccgcagcaccagcgattgcggcggcccgaaagatcatccgctgacctgcgatcatccgcagctgagcggcctgctgtttctg

SEQ ID NO. 54: nucleotide sequence of wild-type human thyroid-stimulating hormone (hTSH) β subunit:

ttttgcattccgaccgaatataccatgcatattgaacgccgcgaatgcgcgtattgcctgaccattaacaccaccatttgcgcgggctattgcatgacccgcgatattaacggcaaactgtttctgccgaaatatgcgctgagccaggatgtgtgcacctatcgcgattttatttatcgcaccgtggaaattccgggctgcccgctgcatgtggcgccgtattttagctatccggtggcgctgagctgcaaatgcggcaaatgcaacaccgattatagcgattgcattcatgaagcgattaaaaccaactattgcaccaaaccgcagaaaagctatctggtgggctttagcgtg

SEQ ID NO. 55: nucleotide sequence of wild-type human follicle-stimulating hormone (hFSH) β subunit:

aacagctgcgaactgaccaacattaccattgcgattgaaaaagaagaatgccgcttttgcattagcattaacaccacctggtgcgcgggctattgctatacccgcgatctggtgtataaagatccggcgcgcccgaaaattcagaaaacctgcacctttaaagaactggtgtatgaaaccgtgcgcgtgccgggctgcgcgcatcatgcggatagcctgtatacctatccggtggcgacccagtgccattgcggcaaatgcgatagcgatagcaccgattgcaccgtgcgcggcctgggcccgagctattgcagctttggcgaaatgaaagaa

SEQ ID NO. 56: nucleotide sequence of wild-type human chorionic gonadotropin (hCG) β1 subunit:

agcaaagaaccgctgcgcccgcgctgccgcccgattaacgcgaccctggcggtggaaaaagaaggctgcccggtgtgcatt

accgtgaacaccaccatttgcgcgggctattgcccgaccatgacccgcgtgctgcagggcgtgctgccggcgctgccgcaggtggt gtgcaactatcgcgatgtgcgctttgaaagcattcgcctgccgggctgcccgcgcggcgtgaacccggtggtgagctatgcggtggc gctgagctgccagtgcgcgctgtgccgccgcagcaccaccgattgcggcggcccgaaagatcatccgctgacctgcgatgatccgc gctttcaggcgagcagcagcagcaaagcgccgccgccgagcctgccgagcccgagccgcctgccgggcccgagcgataccccg attctgccgcag

SEQ ID NO. 57: nucleotide sequence of wild-type human chorionic gonadotropin (hCG) β2 subunit:

agcaaagaaccgctgcgcccgcgctgccgcccgattaacgcgaccctggcggtggaaaaagaaggctgcccggtgtgcatt accgtgaacaccaccatttgcgcgggctattgcccgaccatgacccgcgtgctgcagggcgtgctgccggcgctgccgcaggtggt gtgcaactatcgcgatgtgcgctttgaaagcattcgcctgccgggctgcccgcgcggcgtgaacccggtggtgagctatgcggtggc gctgagctgccagtgcgcgctgtgccgccgcagcaccaccgattgcggcggcccgaaagatcatccgctgacctgcgatgatccgc gctttcaggatagcagcagcagcaaagcgccgccgccgagcctgccgagcccgagccgcctgccgggcccg

SEQ ID NO. 58: nucleotide sequence of wild-type human chorionic gonadotropin (hCG) β3 subunit:

agcaaagaaccgctgcgcccgcgctgccgcccgattaacgcgaccctggcggtggaaaaagaaggctgcccggtgtgcatt accgtgaacaccaccatttgcgcgggctattgcccgaccatgacccgcgtgctgcagggcgtgctgccggcgctgccgcaggtggt gtgcaactatcgcgatgtgcgctttgaaagcattcgcctgccgggctgcccgcgcggcgtgaacccggtggtgagctatgcggtggc gctgagctgccagtgcgcgctgtgccgccgcagcaccaccgattgcggcggcccgaaagatcatccgctgacctgcgatgatccgc gctttcaggatagcagcagcagcaaagcgccgccgccgagcctgccgagcccgagccgcctgccgggcccgagcgataccccga ttctgccgcag

SEQ ID NO. 59: nucleotide sequence of wild-type human chorionic gonadotropin (hCG) β5 subunit:

agccgcgaaatgctgcgcccgcgctgccgcccgattaacgcgaccctggcggtggaaaaagaaggctgcccggtgtgcatt accgtgaacaccaccatttgcgcgggctattgcccgaccatgacccgcgtgctgcagggcgtgctgccggcgctgccgcaggtggt gtgcaactatcgcgatgtgcgctttgaaagcattcgcctgccgggctgcccgcgcggcgtgaacccggtggtgagctatgcggtggc gctgagctgccagtgcgcgctgtgccgccgcagcaccaccgattgcggcggcccgaaagatcatccgctgacctgcgatgatccgc gctttcaggcgagcagcagcagcaaagcgccgccgccgagcctgccgagcccgagccgcctgccgggcccgagcgataccccg attctgccgcag

SEQ ID NO. 60: nucleotide sequence of wild-type human chorionic gonadotropin (hCG) β7 subunit:

agccgcgaaatgctgcgcccgcgctgccgcccgattaacgcgaccctggcggtggaaaaagaaggctgcccggtgtgcatt accgtgaacaccaccatttgcgcgggctattgcccgaccatgacccgcgtgctgcagggcgtgctgccggcgctgccgcaggtggt gtgcaactatcgcgatgtgcgctttgaaagcattcgcctgccgggctgcccgcgcggcgtgaacccggtggtgagctatgcggtggc gctgagctgccagtgcgcgctgtgccgccgcagcaccaccgattgcggcggcccgaaagatcatccgctgacctgcgatgatccgc gctttcaggcgagcagcagcagcaaagcgccgccgccgagcctgccgagcccgagccgcctgccgggcccgagcgataccccg attctgccgcag

SEQ ID NO. 61: nucleotide sequence of mutant human glycoprotein hormone α subunit:

ccgccggatgtgcaggattgcccggaatgcaccctgcaggaaaacccgttttttagccagccgggcgcgccgattctgcagtg catgggctgctgctttagccgcgcgtatccgaccccgctgcgcagcaaaaaaaccatgctggtgcagaaaaacgtgaccagcgaaa gcacctgctgcgtggcgaaaagctataaccgcgtgaccgtgatgggcggctttaaagtggaaaaccataccgcgtgccattgcagca cctgctattatcataaaagc

SEQ ID NO. 62: nucleotide sequence of mutant human glycoprotein hormone α subunit:

cagccggatgtgcaggattgcccggaatgcaccctgcaggaaaacccgttttttagccagccgggcgcgccgattctgcagtg catgggctgctgctttagccgcgcgtatccgaccccgctgcgcagcaaaaaaaccatgctggtgcagaaaaacgtgaccagcgaaa gcacctgctgcgtggcgaaaagctataaccgcgtgaccgtgatgggcggctttaaagtggaaaaccataccgcgtgccattgcagca cctgctattatcataaaagc

SEQ ID NO. 63: nucleotide sequence of mutant human glycoprotein hormone α subunit:

aacccggatgtgcaggattgcccggaatgcaccctgcaggaaaacccgttttttagccagccgggcgcgccgattctgcagtg catgggctgctgctttagccgcgcgtatccgaccccgctgcgcagcaaaaaaaccatgctggtgcagaaaaacgtgaccagcgaaa gcacctgctgcgtggcgaaaagctataaccgcgtgaccgtgatgggcggctttaaagtggaaaaccataccgcgtgccattgcagca cctgctattatcataaaagc

SEQ ID NO. 64: nucleotide sequence of wild-type mouse glycoprotein hormone α subunit:

ctgccggatggcgattttattattcagggctgcccggaatgcaaactgaaagaaaacaaatattttagcaaactgggcgcgccg atttatcagtgcatgggctgctgctttagccgcgcgtatccgaccccggcgcgcagcaaaaaaaccatgctggtgccgaaaaacattac cagcgaagcgacctgctgcgtggcgaaagcgtttaccaaagcgaccgtgatgggcaacgcgcgcgtggaaaaccataccgaatgc cattgcagcacctgctattatcataaaagc

SEQ ID NO. 65: nucleotide sequence of wild-type mouse luteinizing hormone (mLH) β subunit:

agccgcggcccgctgcgcccgctgtgccgcccggtgaacgcgaccctggcggcggaaaacgaattttgcccggtgtgcatt acctttaccaccagcatttgcgcgggctattgcccgagcatggtgcgcgtgctgccggcggcgctgccgccggtgccgcagccggtg tgcacctatcgcgaactggcgtttgcgagcgtgcgcctgccgggctgcccgccgggcgtggatccgattgtgagctttccggtggcgc tgagctgccgctgcggcccgtgccgcctgagcagcagcgattgcggcggcccgcgcacccagccgatggcgtgcgatctgccgca tctgccgggcctgctgctgctg

SEQ ID NO. 66: nucleotide sequence of wild-type mouse thyroid-stimulating hormone (mTSH) β subunit:

ttttgcattccgaccgaatataccatgtatgtggatcgccgcgaatgcgcgtattgcctgaccattaacaccaccatttgcgcggg ctattgcatgacccgcgatattaacggcaaactgtttctgccgaaatatgcgctgagccaggatgtgtgcacctatcgcgattttatttatc gcaccgtggaaattccgggctgcccgcatcatgtgaccccgtattttagctttccggtggcggtgagctgcaaatgcggcaaatgcaac accgataacagcgattgcattcatgaagcggtgcgcaccaactattgcaccaaaccgcagagcttttatctgggcggctttagcgtg

SEQ ID NO. 67: nucleotide sequence of wild-type mouse follicle-stimulating hormone (mFSH) β subunit:

tgcgaactgaccaacattaccattagcgtggaaaaagaagaatgccgcttttgcattagcattaacaccacctggtgcgcgggct attgctatacccgcgatctggtgtataaagatccggcgcgcccgaacacccagaaagtgtgcacctttaaagaactggtgtatgaaacc gtgcgcctgccgggctgcgcgcgccatagcgatagcctgtatacctatccggtggcgaccgaatgccattgcggcaaatgcgatagc gatagcaccgattgcaccgtgcgcggcctgggcccgagctattgcagctttagcgaaatgaaagaa

SEQ ID NO. 68: nucleotide sequence of mutant mouse glycoprotein hormone α subunit:

ccgccggatggcgattttattattcagggctgcccggaatgcaaactgaaagaaaacaaatattttagcaaactgggcgcgccg atttatcagtgcatgggctgctgctttagccgcgcgtatccgaccccggcgcgcagcaaaaaaaccatgctggtgccgaaaaacattac cagcgaagcgacctgctgcgtggcgaaagcgtttaccaaagcgaccgtgatgggcaacgcgcgcgtggaaaaccataccgaatgc cattgcagcacctgctattatcataaaagc

SEQ ID NO. 69: nucleotide sequence of mutant mouse glycoprotein hormone α subunit:

cagccggatggcgattttattattcagggctgcccggaatgcaaactgaaagaaaacaaatattttagcaaactgggcgcgccg atttatcagtgcatgggctgctgctttagccgcgcgtatccgaccccggcgcgcagcaaaaaaaccatgctggtgccgaaaaacattac cagcgaagcgacctgctgcgtggcgaaagcgtttaccaaagcgaccgtgatgggcaacgcgcgcgtggaaaaccataccgaatgc cattgcagcacctgctattatcataaaagc

SEQ ID NO. 70: nucleotide sequence of mutant mouse glycoprotein hormone α subunit:

acccggatggcgattttattattcagggctgcccggaatgcaaactgaaagaaaacaaatattttagcaaactgggcgcgccgat

ttatcagtgcatgggctgctgctttagccgcgcgtatccgaccccggcgcgcagcaaaaaaaccatgctggtgccgaaaaacattacc

agcgaagcgacctgctgcgtggcgaaagcgtttaccaaagcgaccgtgatgggcaacgcgcgcgtggaaaaccataccgaatgcc

attgcagcacctgctattatcataaaagc

## Definitions

In order to provide a clear description of the present invention, a number of terms are defined as follows.

As described herein, a "heteromeric composition", "heteromeric protein", "heterodimeric protein or polypeptide", or "heteromultimeric protein or polypeptide" refers to a protein substance or polypeptide substance, and is a protein or polypeptide composition composed of at least two different amino acid sequences, which may be natural or one or more compositions of proteins or polypeptides expressed by recombinant cells or synthesized by cell-free technology or chemical synthesis technology.

An "interleukin binding protein" refers to a receptor fragment or antibody fragment that can specifically bind to a certain type of interleukin.

A "cytokine binding protein" refers to a receptor fragment or antibody fragment that can specifically bind to a certain type of cytokine.

An "antibody", "monoclonal antibody", and "polyclonal antibody" refer to an immunoglobulin molecule, which is a heterotetrameric protein produced by combining two identical heavy chains and two identical light chains, and specifically bind to an antigen through antigen binding sites composed of light chain variable regions (VL) and heavy chain variable regions (VH), thereby inducing an antigen-specific humoral immune response.

An "antibody fragment" and "antigen-binding peptide" refer to an antibody fragment having antigen-binding ability, and includes fragments produced by cleaving antibodies with protein cleaving enzymes and recombinantly produced single-chain fragments. Examples thereof include Fab, F(ab')2, scFv, diabodies, triabodies, sdAb and VHH.

An "Fc region of immunoglobulin or a part thereof" and "Fc fragment" refer to a protein including the heavy chain constant region 2 (CH2) and the heavy chain constant region 3 (CH3) of an immunoglobulin, but excluding heavy chain variable region and light chain and light chain constant region (CL1) thereof, and may also include the hinge region of the heavy chain constant region, and also a Fc fragment modified on the basis thereof.

A "nucleic acid" or "nucleic acid sequence" refers to a deoxyribonucleotide or ribonucleotide polymer in a single or double-stranded form, and unless otherwise defined, includes known analogs of natural nucleotides that hybridize to nucleic acids in a manner similar to naturally occurring nucleotides. Unless otherwise stated, a specific nucleic acid sequence includes a complementary sequence thereof.

"Mutation" refers to a change in the amino acid sequence encoded by a nucleic acid sequence caused by addition, deletion or substitution in a nucleic acid sequence.

The term "encoding" in relation to a specific nucleic acid includes reference to a nucleic acid containing information for translation into a specific protein. This information is specified by the use of codons.

"Blocking" refers to changing the amino acid type at the first position of an N-terminal by means of mutation, enzyme catalysis, chemical synthesis or the like, resulting in the loss or loss of reactivity of the $\alpha$-amino group that should have existed at the N-terminal, such that it cannot react with an aldehyde derivative or ketone derivative of polyethylene glycol.

An "aldehyde derivative of polyethylene glycol" refers to a compound formed by modifying polyethylene glycol and introducing at least one aldehyde group at any position of polyethylene glycol.

A "ketone derivative of polyethylene glycol" refers to a compound formed by modifying polyethylene glycol and introducing at least one keto group at any position of polyethylene glycol.

"Site-specific single modification" refers to the introduction of a polyethylene glycol molecule at a specific position of a protein.

A "double-end modified derivative" refers to the introduction of active groups at both ends of polyethylene glycol, including at least one aldehyde group or one ketone group, and one other active group. It ensures that one polyethylene glycol molecule can undergo coupling reaction with two different biological macromolecules or small chemical molecules.

"Coupling" and "coupling reaction" refer to the process of obtaining one molecule through a certain chemical reaction between two substance molecules.

"Modification" refers to the connection of one substance to another substance by coupling means or physical means. This connection can be a chemical bond connection or a physical connection.

A "host cell" is a cell that can support the replication or expression of an expression vector. A host cell can be a prokaryotic cell such as Escherichia coli, or a eukaryotic cell such as a yeast cell, an insect cell, an amphibian cell, or a mammalian cell.

As used herein, "polypeptide", "peptide" and "protein" are used interchangeably and include a polymer involving an amino acid residue.

The term "residue" or "amino acid residue" or "amino acid" includes an amino acid that is incoporated into a protein, polypeptide or peptide (collectively referred to as a "peptide"). The amino acid may be a naturally occurring amino acid and, unless otherwise defined, may include known analogs of natural amino acids that can function in a manner similar to naturally occurring amino acids.

"Transfection", "stable transfection" and "transient transfection" refer to the uptake of an expression vector by a host cell, regardless of whether any coding sequence is actually expressed. A variety of transfection methods are known to those skilled in the art. For example, transfection is accomplished in the presence of an expression vector and calcium phosphate at a high concentration, by electroporation, by inserting a bacteriophage or viral expression vector into a host cell, by mechanical insertion of a nucleic acid, or even by culturing a host cell in the presence of an unpackaged nucleic acid fragment. Successful transfection is usually confirmed when any indication of the manipulation of the vector of interest appears in the host cell.

An "expression system" or "expression vector" refers to a nucleic acid sequence containing the desired coding sequence and control sequence in operable connection such that a host transformed with these sequences can produce the encoded protein. To achieve transformation, the expression system can be included on a vector. However, relevant nucleic acid molecules can also be subsequently integrated into a host chromosome.

## Brief Description of Drawings

FIG. 1 is a diagram of a DOT assay of part of products of human modified IL-12.
FIG. 2 is a diagram of a DOT assay of part of products of mouse modified IL-12.
FIG. 3 is an SDS-PAGE diagram of purification results of mP40Q.
FIG. 4 is a bar chart of the expression level of part of mutant human IL-12.
FIG. 5 is a non-reducing gel and reducing SDS-PAGE gels of purified mPEG-pALD-20KD-mP40Q and mPEG-pALD-20KD-mIL-12.
FIG. 6 illustrates the content of mIL-12 in the serum of experimental animals.
FIG. 7 illustrates the content of IFN-$\gamma$ in the serum of experimental animals.
FIG. 8 illustrates the killing ratio of Jurkat cells killed by NK92 cells stimulated by PEG-IL-12-1 and PEG-IL-12-5.
FIG. 9 illustrates the killing ratio of K562 cells killed by NK92 cells stimulated by PEG-IL-12-1 and PEG-IL-12-5.
FIG. 10 illustrates the killing ratio of SK-BR3 cells killed by NK92 cells stimulated by PEG-IL-12-1 and PEG-IL-12-5.
FIG. 11 illustrates the killing ratio of NCI-N87 cells killed by NK92 cells stimulated by PEG-IL-12-1 and PEG-IL-12-5.
FIG. 12 is a graph of non-reducing and reducing SDS-PAGE of purified hCG.
FIG. 13 is a graph of the purification result of PEG-modified natural hCG and hCG-A1P assayed by SDS-PAGE.

## Detailed Description of the Invention

To gain a clearer understanding of the technical features, objects and beneficial effects of the present invention, the following detailed description will be given with reference to specific examples and technical solutions of the present invention, and it should be understood that these examples are merely used to illustrate the present invention and are not used to limit the scope of the present invention. For those skilled in the art, various changes and/or modifications that could have been easily contemplated within the spirit and scope of the present invention are all intended to be included in the protection scope of the present invention. In the examples, all raw reagents and materials are commercially available, and experimental methods without specified conditions are methods and conditions well known in the related art, or according to the conditions recommended by instrument manufacturers.

### Example 1: Mutated IL-12

IL-12 is a heterodimeric cytokine with two subunits, P35 and P40. In the present invention, the N-terminal $\alpha$-amino group of one of the subunits was blocked by gene mutation.

The blocking of the P35 subunit of human IL-12 included mutating the arginine (Arg1) at the first position at the N-terminal of the P35 subunit to proline (Arg1Pro), glutamine (Arg1Gln) or asparagine (Arg1Asn). In this example, the specific amino acid sequence of the mutant human IL-12 P35 subunit can be found in SEQ ID NO. 3, SEQ ID NO. 4, SEQ ID NO. 5.

The blocking of the P40 subunit of human IL-12 includes mutating the isoleucine (Ile1) at the first position at the N-terminal of the P40 subunit to proline (Ile1Pro), glutamine (Ile1Gln) or asparagine (Ile1Asn). In this example, the specific

amino acid sequences of the mutant human IL-12 P40 subunit can be found in SEQ ID NO. 6, SEQ ID NO. 7, SEQ ID NO. 8.

The blocking of the P35 subunit of mouse IL-12 includes mutating the arginine (Arg1) at the first position at the N-terminal of the P35 subunit to proline (Arg1Pro), glutamine (Arg1Gln) or asparagine (Arg1Asn). In this example, the specific amino acid sequences of the mutant mouse IL-12 P35 subunit can be found in SEQ ID Nos. 11 to 13.

The blocking of the P40 subunit of mouse IL-12 includes mutating the methionine (Met1) at the first position at the N-terminal of the P40 subunit to proline (Met1Pro), glutamine (Met1Gln) or asparagine (Met1Asn). In this example, the specific amino acid sequences of the mutant mouse IL-12 P40 subunit can be found in SEQ ID NOs. 14 to 16.

In addition, the IL-12 protein or a fragment thereof may include various modified proteins or peptides, i.e., variants. The above modification may be performed by substituting, deleting or adding at least one amino acid residue of wild-type IL-12 without changing the function of IL-12. These variant proteins or peptides may exhibit 70%, 75%, 80%, 85%, 90%, 91%, 92%, 93%, 94%, 95%, 96%, 97%, 98% or 99% homology to the wild-type protein.

In some embodiments, the variants are obtained by substituting at least one amino acid residue of the wild-type protein without changing the function of the protein. Conventionally, alanine can be substituted for the wild-type amino acid residue, and the substitution can be a conservative amino acid substitution, which has little to no effect on the overall protein charge, i.e., polarity or hydrophobicity. For conservative amino acid substitutions, reference can be made to Table 1.

Table 1

| | |
|---|---|
| Alkaline | Arginine (Arg, R) |
| | Lysine (Lys, K) |
| | Histidine (His, H) |
| Acidic | Glutamic acid (Glu, E) |
| | Aspartic acid (Asp, D) |
| Polar, uncharged | Glutamine (Gln, Q) |
| | Asparagine (Asn, N) |
| | Serine (Ser, S) |
| | Threonine (Thr, T) |
| | Tyrosine (Tyr, Y) |
| Non-polar | Phenylalanine (Phe, F) |
| | Tryptophan (Trp, W) |
| | Cysteine (Cys, C) |
| | Glycine (Gly, G) |
| | Alanine (Ala, A) |
| | Valine (Val, V) |
| | Proline (Pro, P) |
| | Methionine (Met, M) |
| | Leucine (Leu, L) |
| | Norleucine(Nle,O) |
| | Isoleucine(Ile, I) |

For each amino acid, an additional conservative substitution includes a "homolog" of the amino acid. Specifically, a "homolog" refers to an amino acid obtained by inserting a methylene group ($CH_2$) into the β-side chain of the amino acid side chain. Examples of the "homolog" may include homophenylalanine, homoarginine, homoserine, etc., but are not limited thereto. As used herein, the term "IL-12 protein" may be used as a concept including "IL-12 protein and a fragment thereof". Unless otherwise specified, the terms "protein", "polypeptide" and "peptide" may be used as interchangeable concepts.

The nucleic acid sequence corresponding to the amino acid sequence was synthesized by whole gene sequence synthesis technology. For example, the amino acid sequence of the P35 subunit was constructed into a vector pxc17.4, and restriction enzyme cleavage sites were HindIII and EcoRI. The amino acid sequence of the P40 subunit was

constructed into a vector pCDNA3.1+, and restriction enzyme cleavage sites were HindIII and EcoRI. The synthesized vector sequence was optimized according to 293F cells, and restriction enzyme cleavage sites of HindIII, EcoRI, SalI, NotI, PvuI, BamHI, and ScaI were avoided during optimization. After all nucleic acid sequences were optimized, a kozak sequence (GCCACC) and a signal peptide were introduced to the N-terminal, and a TAA termination codon was added to the C-terminal. It should be noted that six His residues were added to the C-terminal of the weakly expressed P35 subunit as a purification tag to reduce the steps and difficulty of purification.

The synthesized vector containing a target gene was transformed and shaken, and the endotoxin-free plasmid was extracted and sequenced. Then, wild-type and modified IL-12 (each modified IL-12 was composed of one P35 subunit and one P40 subunit) were prepared according to the combinations in Table 2, and expression vectors carrying the P35 nucleic acid sequence and the P40 nucleic acid sequence were co-transfected into 293F cells for transient expression. The expression vectors containing P35 and P40 were used in a 1:1 ratio, the concentration of the expression vectors was 0.8 $\mu$g/mL, the transfection reagent was 1.2 $\mu$L/mL, the transfection system was opti-MEM, and the transfection reagent was FectoPRO®. One day before transfection, 500 mL of cell suspension was prepared at 1 x $10^6$ cells/mL. The cells were cultured overnight at 37°C, 125 rpm and 5% $CO_2$. During transfection, the corresponding volume of FectoPRO® was added to a 50 mL empty tube. In a second 50 mL tube, the DNA plasmid with a desired concentration was diluted in serum-free medium to a final volume of 10% of the total volume and gently mixed. The diluted DNA was poured into the pure FectoPRO® reagent at one time, and the solution was immediately mixed and incubated at room temperature for 10 minutes. The FectoPRO®/DNA transfection mixture was poured into the cells and mixed thoroughly with the cell suspension. If necessary, FectoPRO®Booster can be added at a final concentration of 0.5 $\mu$L/mL 2 hours after transfection. After 6 hours, the medium was replaced with serum-free medium from Shanghai OPM Biosciences Co.,Ltd., and the culture was continued. The cells were counted and monitored for 48 hours. When the cell viability was higher than 80%, the medium was supplemented at a ratio of 5% and the culture was continued. Otherwise, the products were harvested and DOT was performed to determine whether the protein was expressed. The test results are illustrated in FIGS. 1 and 2. FIG. 1 illustrates the expression of part of products of human modified IL-12. The darker the black, the more proteins were expressed. It can be seen that combinations 1, 4, 6, and 7 have higher expression levels. FIG. 2 illustrates the expression of part of mouse IL-12, from which it can be seen that combinations 8, 10, and 13 have higher expression levels. The expressed protein was purified by His affinity chromatography column, and the purity and molecular weight were confirmed by SDS-PAGE. FIG. 3 is a diagram of the purification results of mP40Q. In FIG. 3, S in lane 1 is the cell supernatant stock solution of the expressed protein, FT in lane 2 is the flow-through of the purified column, in which most of the target protein has been captured, lane 3 is the collected solution of column washing, which contains only impurities but no target protein, lanes 4 to 8 are samples collected by volume of column eluate, most of which contain the target protein, with 2B3 containing the most target protein, lane 9 is a standard used as a control, and lane 10 is a marker lane used as a reference for molecular weight.

Table 2

| Combination number | Combination name | P35 | P40 |
|---|---|---|---|
| 1 | Wild-type human IL-12 | SEQ ID NO. 1 | SEQ ID NO. 2 |
| 2 | hP35P | SEQ ID NO. 3 | SEQ ID NO. 2 |
| 3 | hP35Q | SEQ ID NO. 4 | SEQ ID NO. 2 |
| 4 | hP35N | SEQ ID NO. 5 | SEQ ID NO. 2 |
| 5 | hP40P | SEQ ID NO. 1 | SEQ ID NO. 6 |
| 6 | hP40Q | SEQ ID NO. 1 | SEQ ID NO. 7 |
| 7 | hP40N | SEQ ID NO. 1 | SEQ ID NO. 8 |
| 8 | Wild-type mouse IL-12 | SEQ ID NO. 9 | SEQ ID NO. 10 |
| 9 | mP35P | SEQ ID No. 11 | SEQ ID NO. 10 |
| 10 | mP35Q | SEQ ID NO. 12 | SEQ ID NO. 10 |
| 11 | mP35N | SEQ ID NO. 13 | SEQ ID NO. 10 |
| 12 | mP40P | SEQ ID NO. 9 | SEQ ID NO. 14 |
| 13 | mP40Q | SEQ ID NO. 9 | SEQ ID NO. 15 |
| 14 | mP40N | SEQ ID NO. 9 | SEQ ID NO. 16 |

A nucleic acid molecule may further include a signal sequence or a leader sequence. As used herein, the term "signal sequence" refers to a fragment that directs the secretion of biologically active molecular drugs and fusion proteins and is cleaved after translation in a host cell. The signal sequence of the present invention is a polynucleotide encoding an amino acid sequence that initiates the movement of proteins that penetrate an endoplasmic reticulum (ER) membrane. Useful signal sequences in the present invention include a signal peptide of a human interleukin-12 P35 subunit, a signal peptide of a human interleukin-12 P40 subunit, a signal peptide of a mouse interleukin-12 P35 subunit, and/or a signal peptide of a mouse interleukin-12 P40 subunit.

The characteristics of signal peptides are well known in the art. Signal peptides generally have 16 to 30 amino acids, but may contain a greater or lesser number of amino acid residues. A signal peptide in the related art consists of three regions: an alkaline N-terminal region, a central hydrophobic region, and a more polar C-terminal region. The central hydrophobic region includes 4 to 12 hydrophobic residues, which fix a signal sequence through the membrane lipid bilayer during the translocation of immature polypeptides. After activation, the signal sequence is usually cleaved in the lumen of the ER by a cellular enzyme called a signal peptidase. Specifically, the signal sequence may be a secretion signal sequence of tissue plasminogen activator (tPa), a signal sequence of herpes simplex virus glycoprotein D (HSV gDs), or a growth hormone. Preferably, a secretion signal sequence used in higher eukaryotic cells including mammals and the like can be used. In addition, as the secretion signal sequence, the signal sequence in wild-type IL-7 can be used, or its codons can be replaced with those with a high expression frequency in the host cell before it is employed.

The expression levels of part of mutant human IL-12 in this example are illustrated in FIG. 4. It can be seen that the expression levels of different mutations are different. The expression level of P35 mutation is significantly lower than that of P40 mutation. The expression levels of different mutation forms of the N-terminal amino acid of P40 are also different. The expression level of hP40P is the highest, followed by hP40Q.

The obtained hP40Q, hP35Q, mP40Q, and mP35Q proteins were diluted to 2 mg/mL with ultrapure water having a pH of 6.0, and then 28 μL of glutamine cyclase with a concentration of 1 ng/mL was added. The proteins were incubated at 37°C for 5 hours to convert the glutamine at the N-terminal of the IL-12 into pyroglutamic acid. After the incubation, the proteins were purified again using a His affinity chromatography column, and proteins with N-terminal α-amino group blocked were obtained and used for the subsequent coupling reaction with an aldehyde derivative of polyethylene glycol.

**Example 2**

This example provides the synthesis of mouse interleukin-12 mP40Q and mP35Q, which are subjected to site-specific single modification using monodisperse 20KD monomethoxy polyethylene glycol propionaldehyde (mPEG-pALD-20K). The specific preparation method is as follows.

(1) Dissolve 5 mg of mouse interleukin-12 mP40Q or mP35Q in 0.05 M acetic acid/sodium acetate buffer with a pH value of 5.0, and stir to dissolve at 20°C to prepare an interleukin-12 solution with a final concentration of 1.0 mg/mL.
(2) Dissolve 14.3 mg of monomethoxy polyethylene glycol propionaldehyde with a molecular weight of 20 KD (purchased from Xiamen Sinopeg Biotechnology Co., Ltd., Cat. No.: 06020101412) in pure water to obtain an aqueous solution of monomethoxy polyethylene glycol propionaldehyde. Then add this solution dropwise to the interleukin-12 solution. The molar ratio of monomethoxy polyethylene glycol propionaldehyde to the interleukin-12 was 10:1.
(3) 0.135 mg of sodium cyanoborohydride (with a molar ratio of sodium cyanoborohydride to interleukin-12 of 30:1) was dissolved in water to prepare an aqueous solution of sodium cyanoborohydride, which was then added to the interleukin-12 solution and stirred at 20°C for 24 hours. The reaction was monitored by UPLC, as illustrated in Table 1 and FIG. 1.
(4) After the reaction was completed, the product was purified. Specifically, the product was filtered through an ultrafiltration tube with a cut-off flow rate of 30 KD, and then purified by ion exchange chromatography to obtain the purified interleukin-12 modified with monodisperse 20 KD monomethoxy polyethylene glycol propionaldehyde.

The schematic diagram of the reaction process of the preparation method in this example is as follows:

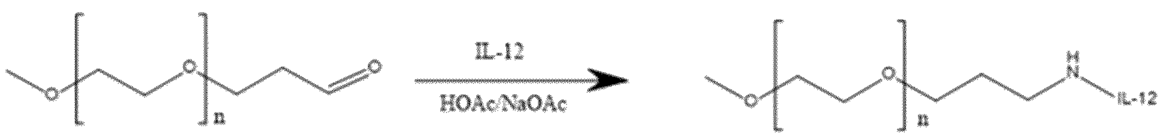


mPEG-pALD-20K

mPEG-pALD-20K-IL-12

The value of n is approximately 454.

In this example, by controlling the above reaction conditions, mPEG-pALD-20KD was selectively site-specifically coupled to the α-amino group at the N-terminal of the P35 subunit of mouse interleukin-12 mP40Q or site-specifically coupled to the α-amino group at the N-terminal of the P40 subunit of mouse interleukin-12 mP35Q.

SDS-PAGE was used to determine the purity of mPEG-pALD-20KD-mP40Q and mPEG-pALD-20KD-mIL-12, and the electrophoresis diagram is shown in FIG. 5. In FIG. 5, lanes 1 and 2 are non-reducing gel and reducing gel results of mPEG-pALD-20KD-mP40Q, respectively, and lanes 3 and 4 are non-reducing gel and reducing gel results of mPEG-pALD-20KD-mIL-12, respectively. The purity of all products is above 95%. By comparing the reducing and non-reducing SDS-PAGE gel images, it can be observed that PEG was predominantly modified on the P35 subunit of mPEG-pALD-20KD-mP40Q, and PEG is also modified on both the P40 and the P35 subunit of mPEG-pALD-20KD-mIL-12. This proves that this method can block the N-terminal α-amino group of one subunit in a heterodimer, enabling an aldehyde derivative of polyethylene glycol to react only with the N-terminal α-amino group of the other subunit. Thus, site-specific single modification of the heterodimer IL-12 can be achieved.

Those skilled in the art can easily extend this technology and use this method for site-specific single modification of other heteromeric proteins or polypeptides with polyethylene glycol.

**Example 3**

An IFN-γ ELISA kit was employed in a control experiment to assess the capacity of mPEG-pALD-20KD-mP40Q and mPEG-pALD-20KD-mP35Q (Example 2) and mIL-12 to stimulate NK-92 cells for IFN-γ production. Subsequently, the activity, difference between mPEG-pALD-20KD-mP40Q and mPEG-pALD-20KD-mP35Q and mIL-12 and the extent of toxicity reduction were evaluated . The specific method is as follows:

(1) The passage number of cultured NK-92 cells was recorded, and the cell status was observed under a microscope. If the status was good, the cells were collected in a 15 mL or 50 mL sterile centrifuge tube and centrifuged at 600 rpm for 5 minutes. The supernatant was discarded, and the cells were resuspended in 5 mL of a fresh complete medium and counted.

(2) Based on the cell counting result, a fresh complete medium was used to dilute the cell suspension to $2\times10^5$ cells/mL, and then the cell suspension was added to a 96-well plate, with 100 μL per well.

(3) The sample was sterilized by filtration and gradient diluted with an NK-92 complete medium to a final concentration of 1000 to 0.46 U/mL. The diluted sample was added to a 96-well plate loaded with 100 μL of cell suspension. With a final culture system of 200 μL, the final cell density was $1\times10^5$ cells/mL, and the cells were stimulated for (24 ± 2) hours.

(4) After stimulation, the 96-well plate was centrifuged at 1000 rpm for 5 minutes. The supernatant was then diluted 60 times with a standard diluent (1X Dilution buffer R) in the ELISA Kit. That is, 5 μL of the supernatant was taken with a 10 μL 8-channel pipette and added to 295 μL of the standard diluent, and then shaken at high speed for 5 minutes using a plate washer. Then, 100 μL of the diluted supernatant was added to the wells of the microplate of the IFN-γ assay kit, and 50 μL of a biotinylated antibody was added, mixed, covered with a sealing film, and incubated at room temperature (18°C to 25°C) for 120 minutes. After incubation, the plate was washed four times with washing solution and patted dry.

(5) Then, 100 μL of streptavidin-HRP was added and incubated at room temperature (18°C to 25°C) for 20 minutes. After incubation, the plate was washed four times with washing solution and patted dry.

(6) 100 μL of TMB was added to each well and incubated at room temperature (18°C to 25°C) in the dark for 5 minutes to 30 minutes. The liquid in the wells gradually turned blue. After incubation at room temperature, 100 μL of the stop solution was added to each well to terminate the reaction. The OD value at 450 nm was measured using a SpectraMax M2 microplate reader.

Result processing: The data were processed using GraphPad Prism6 software, with the OD value as the ordinate and dilution multiple as the abscissa. Four parameters were used for fitting to obtain an activity curve, the maximum value(Top value), the minimum value(Bottom value) and the $R^2$ value. The test standard required an $R^2$ value greater than or equal to 0.95. The activity of a test sample was calculated according to the following formula:

$$\text{Biological activity of test sample (U/mL)} = P_r \times \frac{D_s \times E_s}{D_r \times E_r}$$

wherein Pr is the biological activity of a standard, that is, the biological activity of WHO IL-12 standard = $1\times10^4$ U/mL, Ds is the pre-dilution factor of the test sample, Dr is the pre-dilution factor of the standard, Es is the dilution factor of the test sample equivalent to the half maximal effective dose of the standard, and Er is the dilution factor of the half maximal

effective dose of the standard. Specific activity of test sample (U/mg) = test sample titer (U/mL)/test sample concentration (mg/mL).

Test results are shown in Table 3 below.

Table 3

| **Sample name** | **Batch number** | **Sample type** | **Sample titer ($10^6$ U/mL)** | **Sample specific activity ($10^6$ U/mg)** | **Protein content (mg/mL)** |
|---|---|---|---|---|---|
| mIL-12 | 20230110 | Mouse | 0.38 | 0.43 | 0.88 |
| hIL-12 | 20220710 | Human | 12.58 | 12.58 | 1.00 |
| mPEG-pALD-20KD-mP40Q | 20230309 | Mouse | 0.023 | 0.033 | 0.68 |
| mPEG-pALD-20KD-mP35Q | 20230427 | Mouse | 0.051 | 0.069 | 0.741 |

As can be seen from the test results in Table 3, compared with the IL-12 standard, the activity of the product mPEG-pALD-20KD-mP40Q obtained in Example 2 decreased to 1/13 of the original, and the activity of mPEG-pALD-20KD-mP35Q decreased to 1/ 6.2 of the original. The decrease in activity means that to produce the same amount of IFN-γ, PEGylated interleukin-12 requires a larger dose than IL-12. In contrust, at the same dose, PEGylated interleukin-12 has lower side effects, which is benifical for the administration of a larger single dose and maintaining a stable drug concentration in the blood for a longer period of time without causing greater side effects. The dose of PEGylated interleukin-12 used in subsequent pharmacodynamic tests was determined based on referring to the test results obtained in Example 3.

**Example 4**

This example presents an analytical experiment to assess the effect of the duration of the drug's action of mPEG-pALD-20KD-mP40Q prepared in Example 2 in normal mice. By conducting a comparison of the duration of action between mPEG-pALD-20KD-mP40Q prepared in Example 2 and wild-type mIL-12 in normal mice, the prolonging effect of PEGylation on long-acting interleukin-12 regarding the drug's action duration can be confirmed.

Four-week-old C57BL/6 normal mice were randomly divided into 5 groups according to their body weight. Two mice in each group were subcutaneously administered a test substance for normal mouse once. The dosing regime for each group is presented in Table 4. Whole blood samples were collected from the tail vein at 6, 12, 24, 72, 120, 192 and 264 hours, with 100 μL collected each time. The blood was collected into sterile EP tubes, left to stand at room temperature for 30 minutes, and serum was obtained by centrifugation at 3000 g for 10 minutes. Interleukin-12 and IFN-γ contained in the serum of each group were quantified by ELISA.

Table 4

| Group | Drug | Dosage (ng/mouse) | Dosage period | Number of animals (n) |
|---|---|---|---|---|
| Solvent control group (model group) | - | - | 1W | 2 |
| mIL-12 group | mIL-12 | 100 | 1W | 2 |
| | | 200 | | 2 |
| | | 400 | | 2 |
| PEG-IL-12 | mPEG-pAL-D-20K-D-mP40Q | 2600 | 1W | 2 |
| | | 6500 | | 2 |
| | | 13000 | | 2 |

The results are shown in FIGS. 6 and 7. The results show that interleukin-12 in the serum of the PEG-IL-12 groups could still be detected at 120 hours, interleukin-12 in the serum of the natural mIL-12 high-dose group could be detected at 24 hours, and interleukin-12 in the serum of the medium- and low-dose groups could only be detected at 12 hours and

could not be detected thereafter. This indicates that PEGylation can prolong the half-life of interleukin-12. In the detection of IFN-$\gamma$, it was observed that the PEGylated interleukin-12 groups produce significantly more IFN-$\gamma$ than the natural mIL-12 groups. The natural mIL-12 groups reached a peak at 24 hours and subsequently decreased to normal levels, indicating that the response of IFN-$\gamma$ was delayed compared with that of interleukin-12. In the PEGylated interleukin-12 groups, except for the high-dose group, which exhibited a decrease, the medium- and low-dose groups still showed an increase at 264 hours, with a more pronounced delay. In general, PEGylation can prolong the half-life of interleukin-12 and provide a sustained therapeutic effect.

**Example 5**

In this example, the same method as in Example 2 was used to modify human interleukin-12 with mPEG-bALD-20KD, obtaining hP40Q (PEG-IL-12-5) and hP35Q (PEG-IL-12-1), which were used for in vitro stimulation of NK92 cells in tumor killing tests.

The density of starved NK92 cells was adjusted to $1\times10^5$/mL, divided into eight portions, and plated in 6-well plates with 1.5 mL per well. mPEG-bALD-20K-hP40Q was prepared at concentrations of 0.088 nmol/L, 0.88 nmol/L, and 8.8 nmol/L, mPEG-bALD-20K-hP35Q was prepared at concentrations of 0.132 nmol/L, 1.32 nmol/L, and 13.2 nmol/L.hIL-12 was prepared at a concentration of 0.142 nmol/L. They were added to the above 6-well plates, stimulated at 37°C and 5% $CO_2$ for 48 hours. A group without drug addition was set as a control group. Subsequently the stimulated NK92 cells were harvested, centrifuged at 1300 r/min for 5 minutes, and resuspended in a medium to a cell density of $1\times10^6$/mL. The cell densities of tumor cells Jurkat, K562, SK-BR3, and NCI-N87 were adjusted to $2\times10^5$/mL and plated in 96-well plates with 50 $\mu$L per well. The stimulated NK92 cells were then added for co-culture at ratios of NK92:Jurkat = 5:1, NK92:K562 = 30:1, NK92:SK-BR3 = 40:1, and NK92:NCI-N87 = 30:1, and a NK92 natural death group and a medium blank control group were set up. After 24 hours of co-culture, a CCK8 kit was used to test and analyze the tumor killing ability.

The tumor killing ability of NK92 cells stimulated by PEG-IL-12-1 and PEG-IL-12-5 is illustrated in FIGs. 8 to 11. FIG. 8 illustrates the killing ratio of Jurkat cells killed by NK92 cells stimulated by PEG-IL-12-1 and PEG-IL-12-5. The tumor killing ratios of the low-dose concentration groups of PEG-IL-12-1 and PEG-IL-12-5 were lower than those of the IL-12 group. The killing ratios of the medium-dose concentration group and the high-dose concentration group were higher than that of IL-12, but only PEG-IL-12-1 showed statistical significance. FIG. 9 illustrates the killing ratio of K562 cells killed by NK92 cells stimulated by PEG-IL-12-1 and PEG-IL-12-5. The results are similar to those of the Jurkat cells. The killing ratios of the medium-dose concentration group and the high-dose concentration group are higher than those of IL-12 group, and there were significant differences between PEG-IL-12-1 and PEG-IL-12-5. FIGs. 10 and 11 illustrate the killing ratios of SK-BR3 cells and NCI-N87 cells killed by NK92 cells stimulated by PEG-IL-12-1 and PEG-IL-12-5, respectively. In the killing test of solid tumors where a higher effect-target ratio was required, the killing effect of the medium-dose concentration group becsme less significant, and only the high-dose concentration group showed a significantly better killing ability than the IL-12 group.

**Example 6: Gene mutations in follicle-stimulating hormone, luteinizing hormone, and chorionic gonadotropin**

Follicle-stimulating hormone, luteinizing hormone, and chorionic gonadotropin are heterodimeric proteins sharing the same $\alpha$-subunit, the glycoprotein hormone $\alpha$ subunit, but having different $\beta$-subunits. The N-terminal $\alpha$-amino group of the $\alpha$-subunit is blocked through gene mutation in the present invention, which enables site-specific modification of the three proteins.

The blocking of the $\alpha$ subunit of the human glycoprotein hormone includes mutating the alanine (Ala1) at the first position at the N-terminal to proline (Ala1Pro), glutamine (Ala1Gln) or asparagine (Ala1Asn). In this example, the specific amino acid sequences of the mutant $\alpha$ subunit of human glycoprotein hormone are shown in SEQ ID NO. 26, SEQ ID NO. 27, SEQ ID NO. 28.

The blocking of the $\alpha$ subunit of mouse glycoprotein hormone includes mutating the leucine (Leu1) at the first position of the N-terminal to proline (Leu1Pro), glutamine (Leu1Gln) or asparagine (Leu1Asn). In this example, the specific amino acid sequences of the mutant $\alpha$ subunits of human glycoprotein hormone are shown in SEQ ID NO. 33, SEQ ID NO. 34, SEQ ID NO. 35.

In addition, follicle-stimulating hormone, luteinizing hormone, chorionic gonadotropin proteins or fragments thereof may include various modified proteins or peptides, i.e., variants. The above-mentioned modification may be performed by substituting, deleting or adding at least one protein of wild-type follicle-stimulating hormone, luteinizing hormone, chorionic gonadotropin without changing the function of follicle-stimulating hormone, luteinizing hormone, chorionic gonadotropin. These different proteins or peptides may exhibit 70%, 75%, 80%, 85%, 90%, 91%, 92%, 93%, 94%, 95%, 96%, 97%, 98% or 99% homology with the wild-type protein.

Conventionally, alanine is used to replace the wild-type amino acid residue. However, the substitution can be a conservative amino acid substitution, which has either no effect or a weak effect on the overall charge of the protein, that is,

its polarity or hydrophobicity. For conservative amino acid substitutions, reference can be made to Table 1.

For each amino acid, an additional conservative substitution includes a "homolog" of the amino acid. Specifically, a "homolog" refers to an amino acid obtained by inserting a methylene group ($CH_2$) into the β-side chain of the amino acid side chain. Examples of the "homolog" may include homophenylalanine, homoarginine, homoserine, etc., but are not limited thereto. As used herein, the term "follicle-stimulating hormone, luteinizing hormone, and chorionic gonadotropin proteins" may be used as a concept including "follicle-stimulating hormone, luteinizing hormone, and chorionic gonadotropin proteins and fragments thereof". Unless otherwise specified, the terms "protein", "polypeptide" and "peptide" may be used as interchangeable concepts.

The nucleic acid sequence corresponding to the amino acid sequence was synthesized by whole gene sequence synthesis technology. For example, the amino acid sequence of the P35 subunit was constructed into a vector pxc17.4, and restriction enzyme cleavage sites were HindIII and EcoRI. The amino acid sequence of the P40 subunit was constructed into a vector pCDNA3.1+, and restriction enzyme cleavage sites were HindIII and EcoRI. The synthesized vector sequence was optimized according to 293F cells, and restriction enzyme cleavage sites of HindIII, EcoRI, SalI, NotI, PvuI, BamHI, and ScaI were avoided during optimization. After all nucleic acid sequences were optimized, a kozak sequence (GCCACC) and a signal peptide were introduced to the N-terminal, and a TAA termination codon was added to the C-terminal. It should be noted that six His residues were added to the C-terminal of the α subunit as a purification tag to reduce the steps and difficulty of purification.

The synthesized vector containing a target gene was transformed and shaken, and the endotoxin-free plasmid was extracted and sequenced.

Subsequently, the α subunit nucleic acid sequence and the β subunit nucleic acid sequence were combined according to Table 5 to prepare wild-type and mutant follicle-stimulating hormone, luteinizing hormone or chorionic gonadotropin (each mutant hormone was composed of an α subunit and a β subunit), and expression vectors carrying the α subunit nucleic acid sequence and the β subunit nucleic acid sequence were co-transfected into 293F cells for transient expression. The expression vectors containing α subunit and β subunit were used in a 1:1 ratio, the concentration of the expression vectors was 0.8 μg/mL, the transfection reagent was 1.2 μL/mL, the transfection system was opti-MEM, and the transfection reagent was FectoPRO®. One day before transfection, 500 mL of cell suspension was prepared at $1 \times 10^6$ cells/mL. The cells were cultured overnight at 37°C, 125 rpm and 5% $CO_2$. During transfection, the corresponding volume of FectoPRO® was added to a 50 mL empty tube. In a second 50 mL tube, the DNA plasmid with a desired concentration was diluted in serum-free medium to a final volume of 10% of the total volume and gently mixed. The diluted DNA was poured into the pure FectoPRO® reagent at one time, and the solution was immediately mixed and incubated at room temperature for 10 minutes. The FectoPRO®/DNA transfection mixture was poured into the cells and mixed thoroughly with the cell suspension. If necessary, FectoPRO®Booster can be added at a final concentration of 0.5 μL/mL 2 hours after transfection. After 6 hours, the medium was replaced with serum-free medium from Shanghai OPM Biosciences Co.,Ltd., and the culture was continued. The cells were counted and monitored for 48 hours. When the cell viability was higher than 80%, the medium was supplemented at a ratio of 5% and the culture was continued. Otherwise, the products were harvested, and then tested and purified.

Table 5

| Combination number | Combination name | α subunit | β subunit |
|---|---|---|---|
| 1 | Wild-type human luteinizing hormone (hLH) | SEQ ID NO. 17 | SEQ ID NO. 18 |
| 2 | Wild-type human thyroid-stimulating hormone (hTSH) | SEQ ID NO. 17 | SEQ ID NO. 19 |
| 3 | Wild-type human follicle-stimulating hormone (hFSH) | SEQ ID NO. 17 | SEQ ID NO. 20 |
| 4 | Wild-type human chorionic gonadotropin (hCG) | SEQ ID NO. 17 | SEQ ID NO. 21/SEQ ID NO. 22/SEQ ID NO. 23/SEQ ID NO. 24/SEQ ID NO. 25 |
| 5 | Mutant human luteinizing hormone (hLH-A1P) | SEQ ID NO. 26 | SEQ ID NO. 18 |
| 6 | Mutant human luteinizing hormone (hLH-A1Q) | SEQ ID NO. 27 | SEQ ID NO. 18 |
| 7 | Mutant human luteinizing hormone (hLH-A1N) | SEQ ID NO. 28 | SEQ ID NO. 18 |

(continued)

| Combination number | Combination name | α subunit | β subunit |
|---|---|---|---|
| 8 | Mutant human thyroid-stimulating hormone (hTSH-A1P) | SEQ ID NO. 26 | SEQ ID NO. 19 |
| 9 | Mutant human thyroid-stimulating hormone (hTSH-A1Q) | SEQ ID NO. 27 | SEQ ID NO. 19 |
| 10 | Mutant human thyroid-stimulating hormone (hTSH-A1N) | SEQ ID NO. 28 | SEQ ID NO. 19 |
| 11 | Mutant human follicle-stimulating hormone (hFSH-A1P) | SEQ ID NO. 26 | SEQ ID NO. 20 |
| 12 | Mutant human follicle-stimulating hormone (hFSH-A1Q) | SEQ ID NO. 27 | SEQ ID NO. 20 |
| 13 | Mutant human follicle-stimulating hormone (hFSH-A1N) | SEQ ID NO. 28 | SEQ ID NO. 20 |
| 14 | Mutant human chorionic gonadotropin (hCG-A1P) | SEQ ID NO. 26 | SEQ ID NO. 21/SEQ ID NO.22/SEQ ID NO. 23/SEQ ID NO. 24/SEQ ID NO. 25 |
| 15 | Mutant human chorionic gonadotropin (hCG-A1Q) | SEQ ID NO. 27 | SEQ ID NO. 21/SEQ ID NO. 22/SEQ ID NO. 23/SEQ ID NO. 24/SEQ ID NO. 25 |
| 16 | Mutant human chorionic gonadotropin (hCG-A1N) | SEQ ID NO. 28 | SEQ ID NO. 21/SEQ ID NO. 22/SEQ ID NO. 23/SEQ ID NO. 24/SEQ ID NO. 25 |
| 17 | Wild-type mouse luteinizing hormone (mLH) | SEQ ID NO. 29 | SEQ ID NO. 30 |
| 18 | Wild-type mouse thyroid-stimulating hormone (mTSH) | SEQ ID NO. 29 | SEQ ID NO. 31 |
| 19 | Wild-type mouse follicle-stimulating hormone (mFSH) | SEQ ID NO. 29 | SEQ ID NO. 32 |
| 20 | Mutant mouse luteinizing hormone (mLH-L1P) | SEQ ID NO. 33 | SEQ ID NO. 30 |
| 21 | Mutant mouse luteinizing hormone (mLH-L1Q) | SEQ ID NO. 34 | SEQ ID NO. 30 |
| 22 | Mutant mouse luteinizing hormone (mLH-L1N) | SEQ ID NO. 35 | SEQ ID NO. 30 |
| 23 | Mutant mouse thyroid-stimulating hormone (mTSH-L1P) | SEQ ID NO. 33 | SEQ ID NO. 31 |
| 24 | Mutant mouse thyroid-stimulating hormone (mTSH-L1Q) | SEQ ID NO. 34 | SEQ ID NO. 31 |
| 25 | Mutant mouse thyroid-stimulating hormone (mTSH-L1N) | SEQ ID NO. 35 | SEQ ID NO. 31 |
| 26 | Mutant mouse follicle-stimulating hormone (mFSH-L1P) | SEQ ID NO. 33 | SEQ ID NO. 32 |
| 27 | Mutant mouse follicle-stimulating hormone (mFSH-L1Q) | SEQ ID NO. 34 | SEQ ID NO. 32 |
| 28 | Mutant mouse follicle-stimulating hormone (mFSH-L1N) | SEQ ID NO. 35 | SEQ ID NO. 32 |

A nucleic acid molecule may further include a signal sequence or a leader sequence. As used herein, the term "signal sequence" refers to a fragment that directs the secretion of biologically active molecular drugs and fusion proteins and is cleaved after translation in a host cell. The signal sequence of the present invention is a polynucleotide encoding an

amino acid sequence that initiates the movement of proteins that penetrate an endoplasmic reticulum (ER) membrane. Useful signal sequences in the present invention include a signal peptide of a human interleukin-12 P35 subunit, a signal peptide of a human interleukin-12 P40 subunit, a signal peptide of a mouse interleukin-12 P35 subunit, and a signal peptide of a mouse interleukin-12 P40 subunit.

The characteristics of signal peptides are well known in the art. Signal peptides generally have 16 to 30 amino acids, but may contain a greater or lesser number of amino acid residues. A signal peptide in the related art is composed of three distinct regions. These regions include an alkaline N-terminal region, a central hydrophobic region, and a more polar C-terminal region. The central hydrophobic region includes 4 to 12 hydrophobic residues, which fix a signal sequence through the membrane lipid bilayer during the translocation of immature polypeptides. After activation, the signal sequence is usually cleaved in the lumen of the ER by a cellular enzyme called a signal peptidase. Specifically, the signal sequence may be a secretion signal sequence of tissue plasminogen activator (tPa), a signal sequence of herpes simplex virus glycoprotein D (HSV gDs), or a growth hormone. Preferably, a secretion signal sequence used in higher eukaryotic cells including mammals and the like can be used. In addition, as the secretion signal sequence, a signal sequence contained in wild-type hFSH, hLH, hTSH, hCG, mFSH, mLH, and mTSH can be used, or it can be used after being replaced with a codon with a high expression frequency in a host cell.

The transiently expressed hCG protein was purified by His affinity chromatography column, and its purity and molecular weight were confirmed by SDS-PAGE. FIG. 12 show a non-reducing and reducing SDS-PAGE diagram of purified hCG. Lanes 1 and 6 are the non-reducing and reducing results of natural hCG, lanes 2 and 7 are the non-reducing and reducing results of hCG-A1P. Lanes 3 and 8 are the non-reducing and reducing results of hCG-A1Q. Lanes 4 and 9 are the non-reducing and reducing results of hCG-A1N. All of them possess high purity and are suitable for PEGylation.

**Example 7**

This example describes the synthesis of mutant human chorionic gonadotropin (hCG-A1P) that undergoes site-specific single modification with monodisperse 20KD monomethoxy polyethylene glycol propionaldehyde (mPEG-pALD-20K). The site-specific PEGylation to proline (P) is simpler than that to glutamine (Q), because proline can form a ring in its native state without enzymatic catalysis. The purity of the site-specific modified product is also relatively high, and there will be no incomplete cyclization as in the case of catalysis by glutamine cyclase in glutamine, which results in some subunits that should have been blocked are still reactive. The specific preparation method is as follows.

(1) Dissolve 5 mg of mutant human chorionic gonadotropin (hCG-A1P) in 0.05 M acetic acid/sodium acetate buffer with a pH value of 5.0, then stir the solution at 20°C to prepare an interleukin-12 solution with a final concentration of 1.0 mg/mL.

(2) Dissolve 14.3 mg of 20 KD monomethoxy polyethylene glycol propionaldehyde (purchased from Xiamen Sinopeg Biotechnology Co., Ltd., Cat. No.: 06020101412) in pure water to prepare an aqueous solution. Subsequently, add this solution dropwise to the interleukin-12 solution, with the molar ratio of monomethoxy polyethylene glycol propionaldehyde to mutant human chorionic gonadotropin (hCG-A1P) being 10:1.

(3) Dissolve 0.135 mg of sodium cyanoborohydride (with a molar ratio of sodium cyanoborohydride to interleukin-12 of 30:1) in water to prepare its aqueous solution. Then add this solution to the mutant human chorionic gonadotropin (hCG-A1P) solution and stir the mixture at 20°C for 24 hours.

The reaction process of the preparation method of this example is shown as follows:

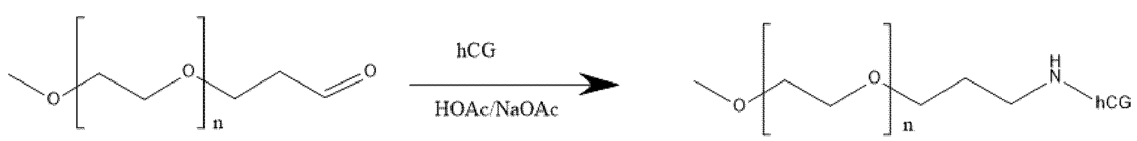


mPEG-pALD-20K

mPEG-pALD-20KD- hCG-A1P

The value of n is approximately 454.

In this example, by controlling the above reaction conditions, mPEG-pALD-20KD was selectively site-specifically coupled to the α-amino group at the N-terminal of the β subunit of human chorionic gonadotropin.

SDS-PAGE was used to test the purity of the purified mPEG-pALD-20KD-hCG-A1P, and the electrophoresis diagram is shown in FIG. 13. In FIG. 13, the purity of all products is above 90%. Lanes 1 and 2 are PEGylated natural hCG. Lanes 3 and 4 are PEGylated HCG-A1P. By comparing the reducing and non-reducing SDS-PAGE gel images, it can be found that PEG can be randomly modified on both subunits of natural hCG, but selectively modified on the β subunit of mPEG-pALD-20KD-hCG-A1P. This proves that this method can block the N-terminal α-amino group of the α subunit of a

heterodimer to allow an aldehyde derivative of polyethylene glycol to only react with the N-terminal α-amino group of β subunit, thereby achieving site-specific single modification of the heterodimer hCG.

The researchers in the field could easily extend this technique and apply this method for site-specific single PEGylation of other heteromeric proteins or polypeptides.

## Claims

1. A method for site-specific PEGylation of a protein or polypeptide, the method comprising:

    a) providing a protein or polypeptide to be modified, the protein or polypeptide to be modified including a target N-terminal α-amino group and a non-target N-terminal α-amino group, and mutating and blocking the non-target N-terminal α-amino group; and
    b) subjecting the target N-terminal α-amino group to a site-specific modification reaction with an aldehyde or ketone derivative of polyethylene glycol to generate a site-specifically PEGylated protein or polypeptide.

2. The method according to claim 1, wherein the protein or polypeptide is a heteromeric protein or polypeptide; preferably, the mutating and blocking the non-target N-terminal α-amino group includes

    constructing a gene encoding an amino acid sequence of the mutated and blocked protein or polypeptide into a vector,
    transforming the vector into a host bacterium and extracting an endotoxin-free plasmid,
    transfecting the plasmid into a host cell to express a target protein, the host cell being preferably a 293F cell, and
    harvesting a target product, which is the protein or polypeptide comprising the mutated and blocked non-target N-terminal α-amino group.

3. The method according to claim 1 or 2, wherein the protein or polypeptide is a heteromeric protein or polypeptide including two to five N-terminal α-amino groups;

    preferably, the heteromeric protein or polypeptide includes one target N-terminal α-amino group and one or two non-target N-terminal α-amino groups,
    preferably, the heteromeric protein or polypeptide with two subunits is selected from one or more of an antibody, a cytokine, a cytokine receptor, a cytokine fusion protein, a hormone, hemoglobin, and a G protein-coupled receptor,
    preferably, the cytokine is selected from one or more of interleukin, interferon, lymphotoxin, a growth factor, and a chemokine,
    preferably, the antibody is selected from a monoclonal antibody, a polyclonal antibody, and an antibody fragment, the antibody fragment preferably including a Fab fragment of an antibody,
    preferably, the hormone is selected from one or more of follicle-stimulating hormone, luteinizing hormone, luteinizing hormone-releasing hormone, thyroid-stimulating hormone, and chorionic gonadotropin, and
    preferably, the interleukin is one or more of interleukin-6, interleukin-12, interleukin-23, interleukin-27 and interleukin-35.

4. The method according to any one of claims 1 to 3, wherein the mutating and blocking the non-target N-terminal α-amino group includes:
mutating the first amino acid at a non-target N-terminal of the protein or polypeptide to be modified into one or more of proline, glutamine, glutamic acid, asparagine, aspartic acid, or an unnatural amino acid by adding, deleting, or substituting one or more amino acid residues.

5. The method according to any one of claims 1 to 4, wherein the aldehyde or ketone derivative of polyethylene glycol is selected from one or more of a polyethylene glycol acetaldehyde derivative, a polyethylene glycol propionaldehyde derivative, and a polyethylene glycol butyraldehyde derivative;
further, the polyethylene glycol propionaldehyde derivative is monomethoxy polyethylene glycol propionaldehyde and/or two-arm polyethylene glycol propionaldehyde.

6. A site-specifically PEGylated protein or polypeptide, wherein a non-PEGylated N-terminal in the amino acid sequence of the protein or polypeptide is mutated and blocked with respect to a wild-type protein or polypeptide;
preferably, the site-specifically PEGylated protein or polypeptide is prepared according to the method according to

any one of claims 1 to 5.

7. The site-specifically PEGylated protein or polypeptide according to claim 6, wherein one end of the polyethylene glycol structure is connected to a target N-terminal of the protein or polypeptide for modification, and the other end of the polyethylene glycol structure is modified with an immunoconjugate or a targeting fragment.

8. The site-specifically PEGylated protein or polypeptide according to claim 6 or 7, wherein the protein or polypeptide has a molecular weight of 5 kD to 40 kD, preferably 10 kD to 30 kD.

9. A medicament comprising:

the site-specifically PEGylated protein or polypeptide according to any one of claims 6 to 8; and a pharmaceutically acceptable excipient;
wherein a dosage form of the medicament includes a liquid preparation and/or a lyophilized preparation;
wherein an administration route of the medicament includes injection, oral administration, topical application, or combined administration via multiple routes.

10. Use of the site-specifically PEGylated protein or polypeptide according to any one of claims 6 to 8 or the medicament according to claim 9 in the manufacture of an anti-tumor medicament.

11. The use according to claim 10, wherein the anti-tumor medicament is administered to a patient alone or in combination with radiation therapy, chemotherapy, myeloablative therapy, CAR-T therapy, CAR-NK therapy, antibody targeted therapy, oncolytic virus therapy and/or gene vaccine therapy.

12. A protein or polypeptide comprising one or more of the following amino acid sequences:
SEQ ID NO. 3, SEQ ID NO. 4, SEQ ID NO. 5, SEQ ID NO. 6, SEQ ID NO. 7, SEQ ID NO. 8; SEQ ID NO. 11, SEQ ID NO. 12, SEQ ID NO. 13, SEQ ID NO. 14, SEQ ID NO. 15, SEQ ID NO. 16, SEQ ID NO. 26, SEQ ID NO. 27, SEQ ID NO. 28, SEQ ID NO. 33, SEQ ID NO. 34, and SEQ ID NO. 35.

13. The protein or polypeptide according to claim 12, which is selected from one or more of the following proteins or polypeptides:

mutant human interleukin-12 with a p35 subunit having an amino acid sequence set forth in any one of SEQ ID NOs. 3 to 5,
mutant human interleukin-12 with a p40 subunit having an amino acid sequence set forth in any one of SEQ ID NOs. 6 to 8,
mutant mouse interleukin-12 with a p35 subunit having an amino acid sequence set forth in any one of SEQ ID NOs. 11 to 13,
mutant mouse interleukin-12 with a p40 subunit having an amino acid sequence set forth in any one of SEQ ID NOs. 14 to 16,
mutant human luteinizing hormone with an $\alpha$ subunit having an amino acid sequence set forth in any one of SEQ ID NOs. 26 to 28 and a $\beta$ subunit having an amino acid sequence set forth in SEQ ID NO. 18,
mutant mouse luteinizing hormone with an $\alpha$ subunit having an amino acid sequence set forth in any one of SEQ ID NOs. 33 to 35 and a $\beta$ subunit having an amino acid sequence set forth in SEQ ID NO. 30,
mutant human thyroid-stimulating hormone with an $\alpha$ subunit having an amino acid sequence set forth in any one of SEQ ID NOs. 26 to 28 and a $\beta$ subunit having an amino acid sequence set forth in SEQ ID NO. 19,
mutant mouse thyroid-stimulating hormone with an $\alpha$ subunit having an amino acid sequence set forth in any one of SEQ ID NOs. 33 to 35 and a $\beta$ subunit having an amino acid sequence set forth in SEQ ID NO. 31,
mutant human follicle-stimulating hormone with an $\alpha$ subunit having an amino acid sequence set forth in any one of SEQ ID NOs. 26 to 28 and a $\beta$ subunit having an amino acid sequence set forth in SEQ ID NO. 20,
mutant mouse follicle-stimulating hormone with an $\alpha$ subunit having an amino acid sequence set forth in any one of SEQ ID NOs. 33 to 35 and a $\beta$ subunit having an amino acid sequence set forth in SEQ ID NO. 32, and
mutant human chorionic gonadotropin with an $\alpha$ subunit having an amino acid sequence set forth in any one of SEQ ID NOs. 26 to 28 and a $\beta$ subunit having an amino acid sequence set forth in any one of SEQ ID NOs. 21 to 25.

14. A nucleic acid molecule encoding the protein or polypeptide according to claim 12 or 13.

15. The nucleic acid molecule according to claim 14, comprising one or more of the following nucleotide sequences:

SEQ ID NO. 38, SEQ ID NO. 39, SEQ ID NO. 40, SEQ ID NO. 41, SEQ ID NO.42, SEQ ID NO. 43, SEQ ID NO. 46, SEQ ID NO. 47, SEQ ID NO.48, SEQ ID NO. 49, SEQ ID NO. 50, SEQ ID NO. 51, SEQ ID NO. 61, SEQ ID NO. 62, SEQ ID NO. 63, SEQ ID NO. 68, SEQ ID NO. 69, and SEQ ID NO.70.

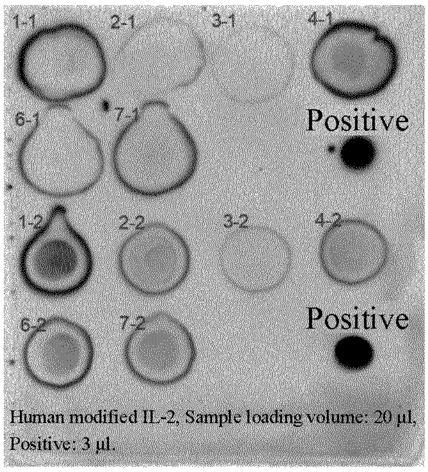


FIG. 1

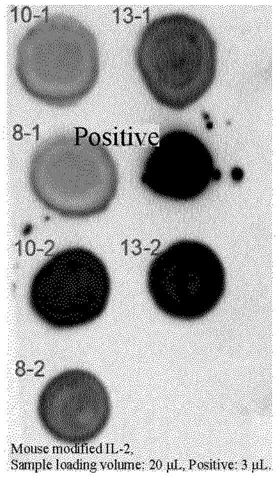


FIG. 2

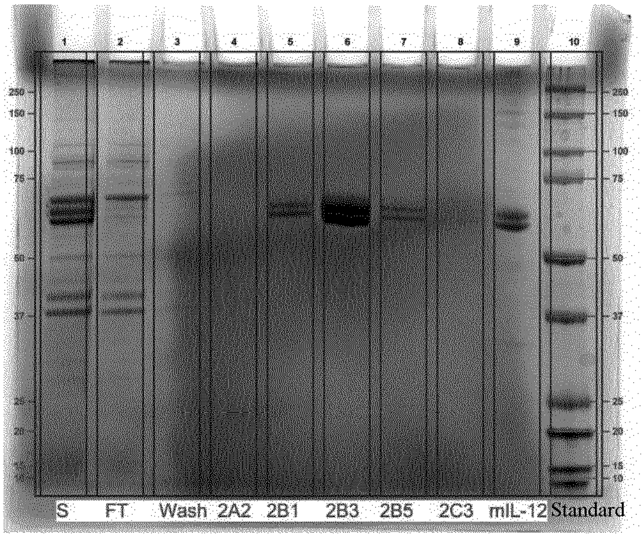


FIG. 3

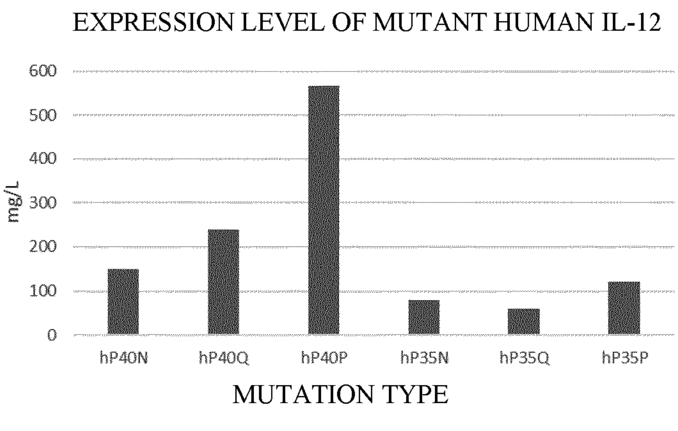


FIG. 4

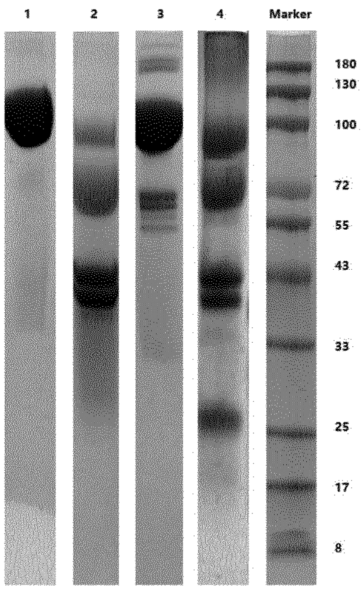


FIG. 5

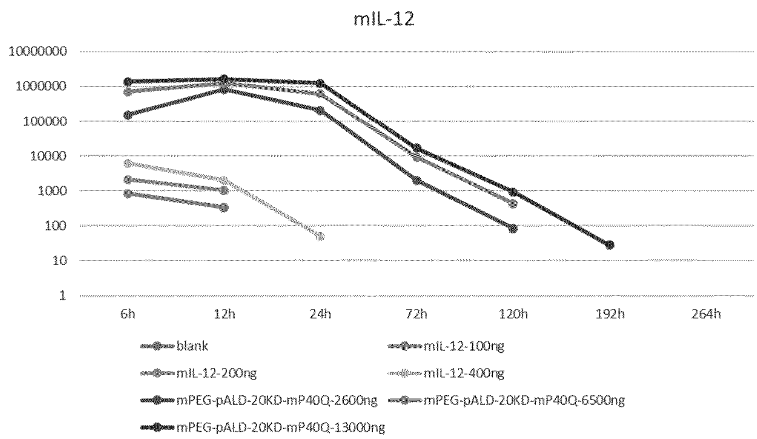


FIG. 6

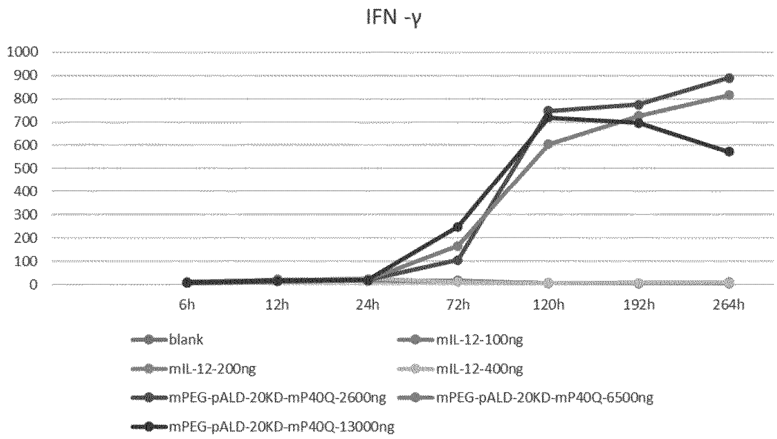


FIG. 7

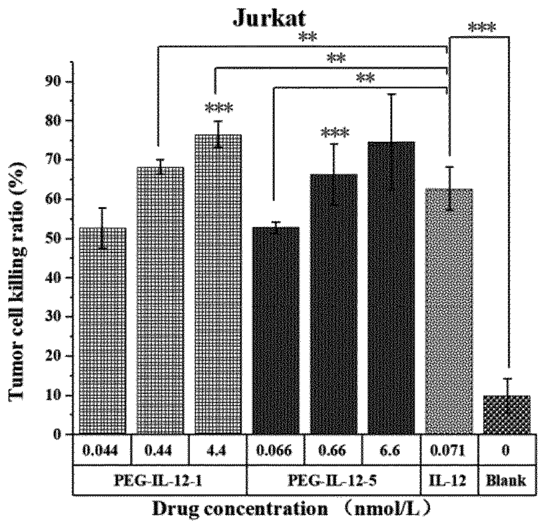


FIG. 8

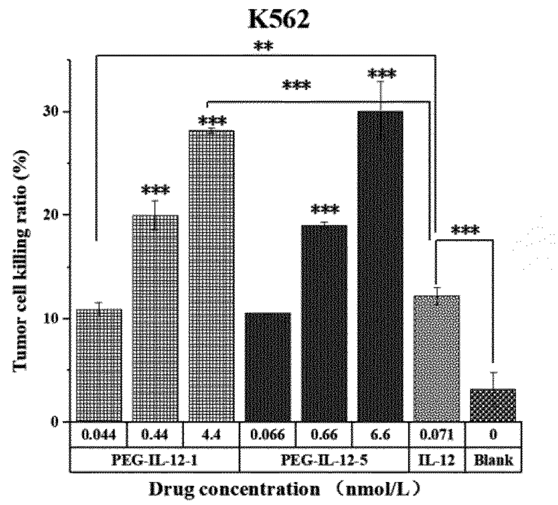


FIG. 9

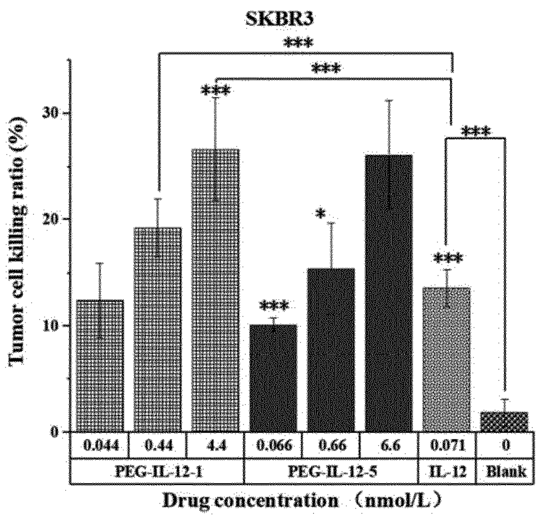


FIG. 10

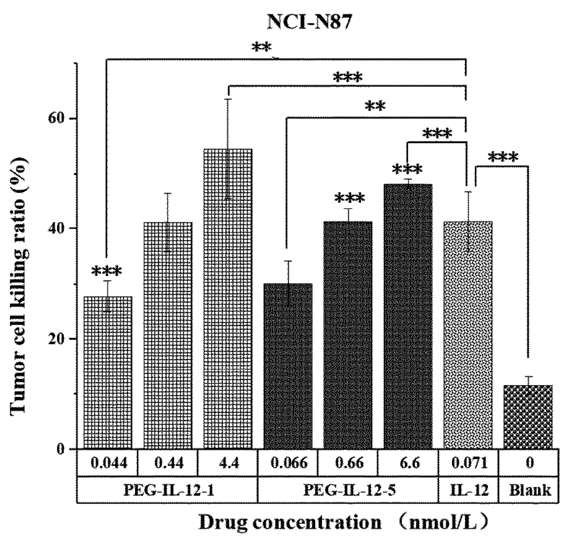


FIG. 11

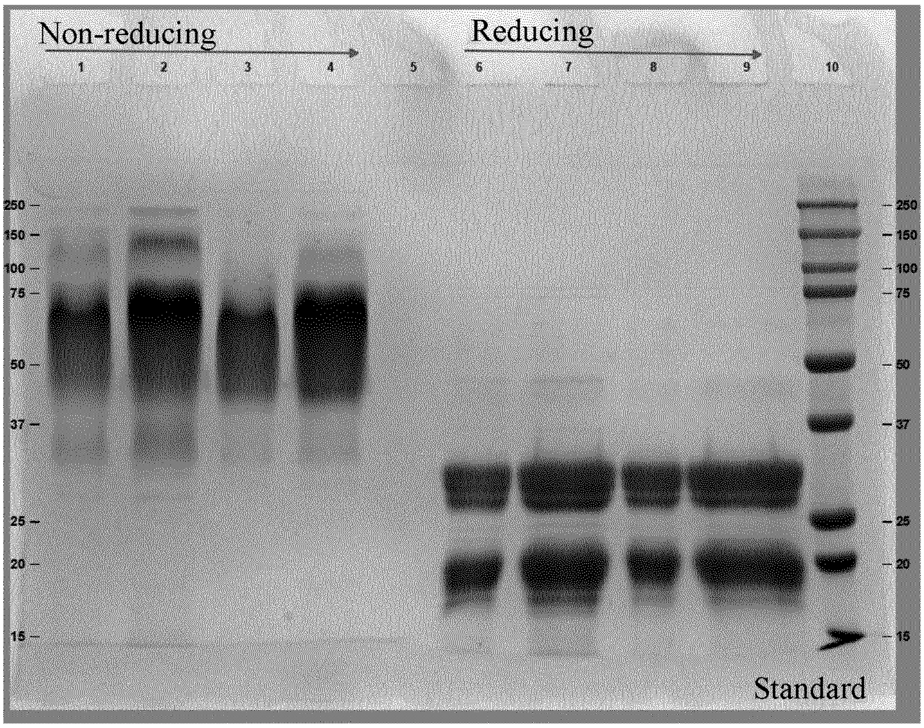
Non-reducing
Reducing
1
2
3
4
5
6
7
8
9
10
250
150
100
75
50
37
25
20
15
Standard

FIG. 12

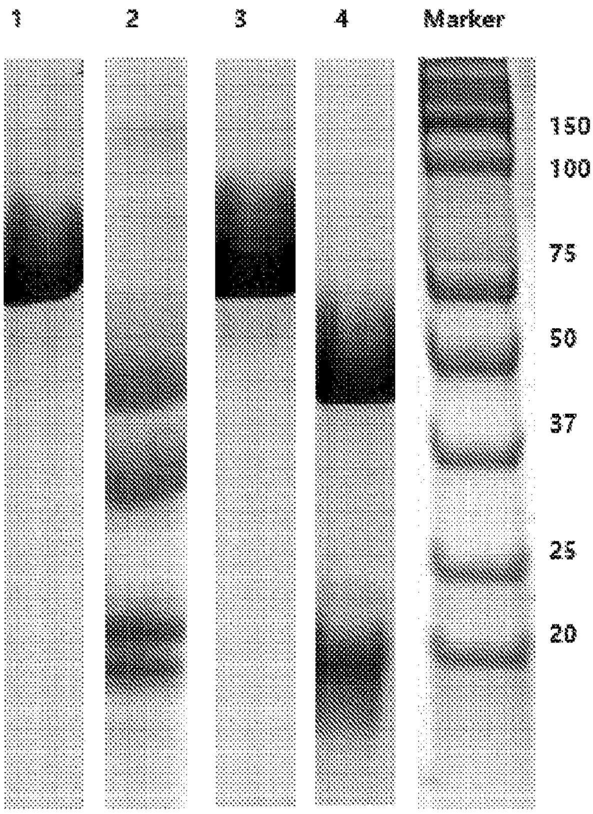
1
2
3
4
Marker
150
100
75
50
37
25
20

FIG. 13

## INTERNATIONAL SEARCH REPORT

International application No.
**PCT/CN2023/131105**

**A. CLASSIFICATION OF SUBJECT MATTER**

C07K1/107(2006.01)i; C07K14/54(2006.01)i; A61K38/20(2006.01)i; A61K47/60(2017.01)i

According to International Patent Classification (IPC) or to both national classification and IPC

**B. FIELDS SEARCHED**

Minimum documentation searched (classification system followed by classification symbols)

IPC:C07K A61K

Documentation searched other than minimum documentation to the extent that such documents are included in the fields searched

Electronic data base consulted during the international search (name of data base and, where practicable, search terms used)

CNTXT; WPABS; ENTXT; USTXT; JPTXT; KRTXT; DWPI; VEN; CJFD; ISI web of Science; PUBMED; 百度学术, BAIDU SCHOLAR; 百度, BAIDU; 读秀, DUXIU; 万方, WANFANG; CNKI; Genbank; EBI; ENA; 中国专利生物序列检索, China Patents Biological Sequence Search System: 聚乙二醇, PEG, 修饰, 偶联, 蛋白, 多肽, 二聚体, 聚体蛋白, 聚体, 非目标, 氮端, N端, a-氨基, 定点修饰, 定点, 选择性, 白介素-12, 白细胞介素12, IL-12, il12, 糖蛋白激素, 亚基, 单修饰, modify, site-specific, N-terminal, protein, target, pegylation, polymer protein, dipolymer, dimer, 序列1-70, sequences 1-70

**C. DOCUMENTS CONSIDERED TO BE RELEVANT**

| Category* | Citation of document, with indication, where appropriate, of the relevant passages | Relevant to claim No. |
|---|---|---|
| A | CN 1970086 A (FUDAN UNIVERSITY) 30 May 2007 (2007-05-30)<br>claims 1-10, and description, page 2, paragraph 2 to page 4, paragraph 1, and page 5, paragraph 5 to page 7, paragraph 4 | 1-15 |
| A | CN 101384620 A (MEDEXGEN CO., LTD.) 11 March 2009 (2009-03-11)<br>sequence 18 | 12-15 |
| A | US 5650492 A (HOFFMANN-LA ROCHE INC.) 22 July 1997 (1997-07-22)<br>sequence 1 | 12-15 |
| A | US 2019169252 A1 (AJOU UNIVERSITY INDUSTRY-ACADEMIC COOPERATION FOUNDATION) 06 June 2019 (2019-06-06)<br>sequence 20 | 12-15 |
| A | US 2007154453 A1 (MERCK PATENT GMBH) 05 July 2007 (2007-07-05)<br>sequence 43 | 12-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2023** | **21 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/131105

C. DOCUMENTS CONSIDERED TO BE RELEVANT

| Category* | Citation of document, with indication, where appropriate, of the relevant passages | Relevant to claim No. |
|---|---|---|
| A | US 6238890 B1 (WASHINGTON UNIVERSITY) 29 May 2001 (2001-05-29)<br>sequence 79 | 12-15 |
| A | US 8530190 B1 (MERCK SERONO SA) 10 September 2013 (2013-09-10)<br>sequence 31 | 12-15 |

Form PCT/ISA/210 (second sheet) (July 2022)

**INTERNATIONAL SEARCH REPORT**

International application No.
**PCT/CN2023/131105**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/131105**

| Patent document cited in search report | | | Publication date (day/month/year) | Patent family member(s) | | | Publication date (day/month/year) |
|---|---|---|---|---|---|---|---|
| CN | 1970086 | A | 30 May 2007 | None | | | |
| CN | 101384620 | A | 11 March 2009 | BRPI | 0412607 | A | 03 July 2007 |
| | | | | CA | 2528458 | A1 | 03 February 2005 |
| | | | | US | 2006008872 | A1 | 12 January 2006 |
| | | | | US | 7569361 | B2 | 04 August 2009 |
| | | | | EP | 1668030 | A4 | 14 June 2006 |
| | | | | EP | 1668030 | A1 | 02 March 2011 |
| | | | | US | 2013089513 | A1 | 11 April 2013 |
| | | | | RU | 2006102846 | A | 10 September 2007 |
| | | | | RU | 2432360 | C2 | 27 October 2011 |
| | | | | DE | 602004031642 | D1 | 14 April 2011 |
| | | | | ES | 2362453 | T3 | 05 July 2011 |
| | | | | ATE | 500266 | T1 | 15 March 2011 |
| | | | | KR | 20050013024 | A | 02 February 2005 |
| | | | | KR | 100632985 | B1 | 11 October 2006 |
| | | | | JP | 2007524403 | A | 30 August 2007 |
| | | | | JP | 4747238 | B2 | 17 August 2011 |
| | | | | WO | 2005010043 | A1 | 03 February 2005 |
| | | | | US | 2010041586 | A1 | 18 February 2010 |
| | | | | ZA | 200600502 | B | 28 February 2007 |
| | | | | AU | 2004259314 | A1 | 03 February 2005 |
| | | | | AU | 2004259314 | B2 | 07 December 2006 |
| US | 5650492 | A | 22 July 1997 | PT | 640689 | E | 31 March 2003 |
| | | | | ES | 2184748 | T3 | 16 April 2003 |
| | | | | ATE | 226215 | T1 | 15 November 2002 |
| | | | | AU | 6607294 | A | 12 January 1995 |
| | | | | AU | 675532 | B2 | 06 February 1997 |
| | | | | RU | 94022238 | A | 27 March 1997 |
| | | | | RU | 2135584 | C1 | 27 August 1999 |
| | | | | ZA | 944567 | B | 27 December 1995 |
| | | | | DK | 0640689 | T3 | 24 February 2003 |
| | | | | ZA | 9404567 | B | 27 December 1995 |
| | | | | DE | 69431545 | D1 | 21 November 2002 |
| | | | | DE | 69431545 | T2 | 26 June 2003 |
| | | | | EP | 0640689 | A2 | 01 March 1995 |
| | | | | EP | 0640689 | A3 | 28 June 1995 |
| | | | | EP | 0640689 | B1 | 16 October 2002 |
| | | | | JPH | 0753594 | A | 28 February 1995 |
| | | | | JP | 3546076 | B2 | 21 July 2004 |
| | | | | NZ | 260843 | A | 27 February 1996 |
| | | | | CA | 2125763 | A1 | 03 January 1995 |
| | | | | CA | 2125763 | C | 28 August 2007 |
| US | 2019169252 | A1 | 06 June 2019 | KR | 20220098108 | A | 11 July 2022 |
| | | | | EP | 3511340 | A1 | 17 July 2019 |
| | | | | EP | 3511340 | A4 | 18 March 2020 |
| | | | | ZA | 201900772 | B | 26 July 2023 |
| | | | | JP | 2021088601 | A | 10 June 2021 |
| | | | | JP | 7111855 | B2 | 02 August 2022 |
| | | | | MX | 2021010809 | A | 01 October 2021 |
| | | | | KR | 20180018419 | A | 21 February 2018 |

Form PCT/ISA/210 (patent family annex) (July 2022)

**INTERNATIONAL SEARCH REPORT**
**Information on patent family members**

International application No.
**PCT/CN2023/131105**

| Patent document cited in search report | Publication date (day/month/year) | Patent family member(s) | Publication date (day/month/year) |
|---|---|---|---|
| | | KR 102050463 B1 | 29 November 2019 |
| | | MX 2019001651 A | 04 September 2019 |
| | | JP 2019536734 A | 19 December 2019 |
| | | JP 6993403 B2 | 03 March 2022 |
| | | AU 2017310163 A1 | 21 March 2019 |
| | | AU 2017310163 B2 | 09 September 2021 |
| | | SG 11201901071 TA | 28 March 2019 |
| | | US 10696722 B2 | 30 June 2020 |
| | | US 2020362004 A1 | 19 November 2020 |
| | | US 11078249 B2 | 03 August 2021 |
| | | CA 3033475 A1 | 15 February 2018 |
| | | BR 112019002394 A2 | 04 June 2019 |
| | | KR 20190134564 A | 04 December 2019 |
| | | KR 102416411 B1 | 05 July 2022 |
| | | JP 2022137288 A | 21 September 2022 |
| | | AU 2021273642 A1 | 16 December 2021 |
| | | US 2020362005 A1 | 19 November 2020 |
| | | US 11692019 B2 | 04 July 2023 |
| US 2007154453 A1 | 05 July 2007 | CY 1112884 T1 | 13 April 2016 |
| | | EA 200870131 A1 | 30 December 2008 |
| | | EA 015338 B1 | 30 June 2011 |
| | | JP 2009521911 A | 11 June 2009 |
| | | JP 5175214 B2 | 03 April 2013 |
| | | BRPI 0620648 A2 | 22 November 2011 |
| | | BRPI 0620648 A8 | 15 January 2019 |
| | | BRPI 0620648 B1 | 20 December 2022 |
| | | PT 1966238 E | 31 July 2012 |
| | | ES 2384055 T3 | 28 June 2012 |
| | | KR 20080090483 A | 08 October 2008 |
| | | KR 101343682 B1 | 20 December 2013 |
| | | ATE 555125 T1 | 15 May 2012 |
| | | AU 2006332138 A1 | 12 July 2007 |
| | | AU 2006332138 B2 | 22 March 2012 |
| | | WO 2007076933 A1 | 12 July 2007 |
| | | SI 1966238 T1 | 31 August 2012 |
| | | ZA 200806611 B | 30 December 2009 |
| | | US 7872107 B2 | 18 January 2011 |
| | | PL 1966238 T3 | 28 September 2012 |
| | | DK 1966238 T3 | 16 July 2012 |
| | | US 2013034518 A1 | 07 February 2013 |
| | | US 9029330 B2 | 12 May 2015 |
| | | CA 2635618 A1 | 12 July 2007 |
| | | CA 2635618 C | 06 October 2015 |
| | | EP 1966238 A1 | 10 September 2008 |
| | | EP 1966238 B1 | 25 April 2012 |
| | | US 2011097792 A1 | 28 April 2011 |
| | | US 8188248 B2 | 29 May 2012 |
| US 6238890 B1 | 29 May 2001 | US 6242580 B1 | 05 June 2001 |
| US 8530190 B1 | 10 September 2013 | JP 2001521402 A | 06 November 2001 |
| | | JP 3950484 B2 | 01 August 2007 |

Form PCT/ISA/210 (patent family annex) (July 2022)

**INTERNATIONAL SEARCH REPORT**
**Information on patent family members**

International application No.
**PCT/CN2023/131105**

| Patent document cited in search report | Publication date (day/month/year) | Patent family member(s) | Publication date (day/month/year) |
|---|---|---|---|
| | | ATE 335000 T1 | 15 August 2006 |
| | | DK 1775346 T3 | 28 September 2009 |
| | | ZA 985545 B | 01 April 1999 |
| | | BR 9810478 A | 12 September 2000 |
| | | KR 20020006720 A | 26 January 2002 |
| | | KR 100560279 B1 | 10 March 2006 |
| | | DE 69835433 D1 | 14 September 2006 |
| | | DE 69835433 T2 | 08 March 2007 |
| | | IL 133686 A | 24 July 2007 |
| | | DK 0996629 T3 | 04 December 2006 |
| | | DE 69841063 D1 | 24 September 2009 |
| | | EP 1775346 A1 | 18 April 2007 |
| | | EP 1775346 B1 | 12 August 2009 |
| | | JP 2005162734 A | 23 June 2005 |
| | | JP 4279768 B2 | 17 June 2009 |
| | | CY 1109439 T1 | 13 August 2014 |
| | | ES 2332931 T3 | 15 February 2010 |
| | | AR 013139 A1 | 13 December 2000 |
| | | KR 20040039421 A | 10 May 2004 |
| | | KR 100706469 B1 | 10 April 2007 |
| | | SI 1775346 T1 | 31 October 2009 |
| | | SI 0996629 T1 | 31 December 2006 |
| | | PT 1775346 E | 25 August 2009 |
| | | HU 0100681 A2 | 28 June 2001 |
| | | HU 0100681 A3 | 01 March 2004 |
| | | HU 227908 B1 | 29 May 2012 |
| | | CA 2293731 A1 | 30 December 1998 |
| | | CA 2293731 C | 12 January 2010 |
| | | ATE 439439 T1 | 15 August 2009 |
| | | JP 2007016040 A | 25 January 2007 |
| | | JP 4278668 B2 | 17 June 2009 |
| | | IL 133686 A0 | 30 April 2001 |
| | | AU 7986198 A | 04 January 1999 |
| | | AU 747179 B2 | 09 May 2002 |
| | | ZA 9805545 B | 01 April 1999 |
| | | PT 996629 E | 29 December 2006 |
| | | WO 9858957 A2 | 30 December 1998 |
| | | WO 9858957 A3 | 22 April 1999 |
| | | WO 9858957 A9 | 03 June 1999 |
| | | EP 0996629 A2 | 03 May 2000 |
| | | EP 0996629 B1 | 02 August 2006 |
| | | HK 1033149 A1 | 17 August 2001 |
| | | ES 2268781 T3 | 16 March 2007 |

Form PCT/ISA/210 (patent family annex) (July 2022)